(19) Europäisches Patentamt
European Patent Office
Office européen des brevets

(11) EP 0 640 747 B1

(12) EUROPEAN PATENT SPECIFICATION

(45) Date of publication and mention of the grant of the patent:
**19.03.1997 Bulletin 1997/12**

(51) Int Cl.[6]: **F01K 21/06**, F22B 37/56, G01N 33/18, C23F 11/08, G05D 21/02

(21) Application number: **94113074.2**

(22) Date of filing: **22.08.1994**

# (54) Boiler system pH/phosphate program control method

System zur Regelung eines Kessels nach einem PH/phosphat-Programm

Système de commande de chaudière par un programme ph/phosphate

(84) Designated Contracting States:
**DE ES FR GB IT**

(30) Priority: **20.08.1993 US 109525**

(43) Date of publication of application:
**01.03.1995 Bulletin 1995/09**

(73) Proprietor: **NALCO CHEMICAL COMPANY**
**Naperville Illinois 60563-1198 (US)**

(72) Inventors:
- **Avallone, Stanley C.**
  **Lockport, Illinois 60441 (US)**
- **Webb, John R.**
  **Napverville, Illinois 60563 (US)**
- **Eisner, Ian E.**
  **Aurora, Illinois 60504 (US)**
- **Fowee, Roger W.**
  **Wheaton, Illinois 60187 (US)**

(74) Representative: **Hartmann, Günter et al**
**RUSCHKE HARTMANN BECKER,**
**Patentanwälte,**
**Pienzenauerstrasse 2**
**81679 München (DE)**

(56) References cited:
**EP-A- 0 320 086**
**EP-A- 0 365 815**
**EP-A- 0 375 326**
**EP-A- 0 385 678**
**GB-A- 2 238 383**
**US-A- 4 709 664**
**US-A- 4 783 314**
**US-A- 5 006 311**
**US-A- 5 019 342**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 640 747 B1

Printed by Jouve, 75001 PARIS (FR)

## Description

### Technical Field of the Invention

The present invention is in the technical field of internal boiler chemical treatment program controls, and in particular the control of pH/phosphate treatment programs.

### Background of the Invention

A boiler is a vessel in which water is vaporized into steam by the application of heat, typically on a continuous basis. The steam generated is most often used either as a direct or indirect heat transfer medium and/or to generate electric power. High pressure and/or high capacity boilers generally are water-tube boilers in which water is circulated within tubes and the applied heat (combustion products such as flame and hot combustion gases) flows across the outside of the tubes. Some of these water tubes may comprise the walls of the furnace in which the heat-generating combustion occurs.

A boiler is a dynamic water system. Essentially pure $H_2O$ leaves the boiler as steam. Water also leaves the boiler as blowdown. The water lost from the boiler as steam or blowdown is replenished with boiler feedwater.

A boiler's internal surfaces are metallic heat transfer surfaces. The formation of scale deposits on, and the corrosion of, these internal surfaces are undesirable phenomena in any type of boiler. Measures taken against scale deposits and/or corrosion depend on the characteristics of the boiler and the boiler feedwater. The preventative measures are generally the purification of the feedwater, internal chemical treatment(s) of the water and the discharge of impurities in the blowdown. The major remedial measure is costly downtime for the cleaning of the internal surfaces. Scale deposition decreases heat transfer rates and promotes corrosion and overheating of these surfaces, and thus control of scale deposition is the primary objective of internal chemical treatment programs such as phosphate residual programs (with and without polymer and/or dispersants), all-polymer treatment programs, chelation programs and coagulation (carbonate) programs. These treatment programs provide preferential precipitation and/or solubilization/dispersion of calcium species and magnesium species.

Some boilers require the use of feedwater having a high degree of purity to ensure high operational reliability. The impurities or chemical species that might be present in boiler feedwater are removed with the use of demineralizers, mixed bed polishers or other techniques in order to purify the feedwater prior to introduction into the boiler. The purity standards allow a maximum feedwater hardness of only 0.1 ppm or less. Such boilers are often, but not necessarily, high pressure, high heat transfer rate boilers that can tolerate almost no solids internally. Boilers operating at pressures at or below 800 psi (which is usually considered a borderline between industrial medium-pressure and high-pressure boilers) may also employ high purity feedwaters. When a boiler's feedwater routinely meets such high purity standards, the internal chemical program of choice is normally a pH/phosphate control program (sometimes known as a coordinated or congruent phosphate control program), optionally together with a dispersant treatment. A pH/phosphate program differs from a standard or residual phosphate program in that the phosphate is primarily added to provide a controlled pH range in the boiler water to provide a buffering counteraction against acid and caustic attack of the internal metallic surfaces, which is described in more detail below. A pH/phosphate control program is very difficult and time consuming to control. The conventional control of such a program is normally based on many assumptions about feedwater phosphate demand, actual boiler cycles and boiler feedwater sodium levels. Feedwater phosphate demand, as that terminology is used herein and generally understood in the boiler field, is phosphate that becomes insoluble within a boiler system, for instance upon the formation of insoluble phosphate species upon combination with calcium or iron ions, or which hides-out (discussed elsewhere) during system load transients. Feedwater phosphate demand is normally a fairly steady value in a well controlled boiler system.

It is an object of the present invention to provide a pH/phosphate control method that is both cost efficient and relatively simple to operate. It is an object of the present invention to provide a pH/phosphate program control process of great sensitivity that is independent of fluctuations in a boiler's steaming rate. It is an object of the present invention to provide a pH/phosphate program control method of great sensitivity that is independent of small impurity variations in the boiler feedwater. It is an object of the present invention to provide such a pH/phosphate program control method for boilers having an operating pressure of at least 15 psi. These and other objects of the present invention are described further below.

### Disclosure of the Invention

The present invention provides a method of controlling a pH/phosphate program in a boiler water system wherein steam is generated from the water within the boiler, feedwater of high purity is fed to the boiler to replenish the water which leaves the boiler as steam and blowdown, and the concentration of impurities in the boiler water is reduced by

withdrawing fractions thereof as blowdown. Such a boiler has a concentration cycle value (boiler cycles), which is the value of the average concentration ("$C_F$") of an inert component in the blowdown at steady state divided by its concentration ("$C_I$") in the feedwater. Under a pH/phosphate treatment program, phosphate is fed to the boiler in amounts to provide a sodium to phosphate ratio in the boiler water within a target ratio range. The method of the present invention controls a pH/phosphate program by employing a plurality of monitorings to obtain crucial data from which the feedwater phosphate concentration, actual boiler cycles and boiler feedwater sodium levels are determined, and integrating the information therefrom. The present invention provides a means of addressing these three parameters (feedwater phosphate concentration, actual boiler cycles and boiler feedwater sodium levels), which are root causes of system variation. The present invention thereby makes it easier to stay within reasonable control parameters with a pH/phosphate program and enhances water treatment chemical control. This leads to a more effective pH/phosphate program and thus higher boiler reliability and lower maintenance costs.

This invention uses:

(a) an inert tracer to determine boiler cycles and to set a correct phosphate feed target as well as to control blowdown rate;
(b) an inert tracer to measure phosphate feed rate, which together with the boiler cycles determination simplify the determination of the residual phosphate level in the boiler; and in some embodiments also
(c) feedwater sodium analysis, which together with the alkalinity feed control (via the introduction of an $OH^-$-alkalinity source, preferably NaOH, with the feedwater) permit a constant hydrate alkalinity level to be maintained in the boiler feedwater, instead of having a varying feed rate that is a fluctuating value based on pretreatment control as an input to the pH/phosphate control box.

The inert tracer overcomes problems with phosphate "hideout", which causes operators to make incorrect program adjustments in their efforts to stay within their control parameter range ("control box"), which is discussed in more detail below.

## Brief Description of the Drawings

FIGURE 1 is a boiler system having monitoring and control means of the present invention shown in diagrammatic form.

FIGURE 2 is a graphical record involving of continuous blowdown tracer monitoring and boiler cycles during time period units T-1 through T-6.

FIGURE 3 is a pH/phosphate control curve.

## Preferred Embodiments of the Invention

The preferred embodiments of the present invention include preferred embodiments concerning monitoring the feedwater phosphate concentration and determining actual boiler cycles, monitoring the feedwater phosphate concentration, determining actual boiler cycles and monitoring boiler feedwater alkalinity levels, and preferred embodiments concerning the pH/phosphate program control system in its entirety and integration of data.

## Caustic-Gouging Corrosion and pH/Phosphate Programs

As noted above, when high purity standards govern feedwater quality, the internal water treatment program of choice for control of scale deposition and corrosion within the boiler system is most often a pH/phosphate program. Stringent feedwater standards are commonly employed for high pressure boilers, but not limited thereto.

High pressure boilers are no longer restricted to utilities. The increasing cost of energy is making cogeneration much more popular. The petrochemical, paper, and chemical industries now commonly use 1200-1800 psig boilers to provide cogeneration of both electricity and steam. Condensing and backpressure turbines are used to drive generators, compressors, and the like, while supplying extraction or exhaust steam for plant use. Waste-heat boilers receive heat from process streams for the production of steam. These high pressure cogenerative and waste-heat boilers have feedwater quality standards at least substantially comparable to that of high pressure utility boilers. In many cases, the initial design of high pressure waste-heat boilers may require even stricter feedwater standards than a utility boiler of higher pressure.

As boiler pressures and heat transfer rates have increased, water-side caustic corrosion of boiler tubes (caustic gouging) has increasingly become a problem. Caustic gouging, or ductile gouging, starts to occur when caustic is allowed to concentrate against hot boiler metal surfaces, dissolving the normally protective magnetite. Normal boiler water hydrate alkalinity levels are harmless to boiler steel, but localized concentrations of tens of thousands of ppm

are very aggressive. At such points, the protective magnetite on the water-side surface of the boiler tube wall is dissolved, as shown in the following Equation 1.

$$\text{Equation 1} \quad \underset{\text{(Caustic)}}{4\,NaOH} + \underset{\text{(Magnetite)}}{Fe_3O_4} \longrightarrow \underset{\text{(Soluble Complexes)}}{2\,NaFeO_2 + Na_2FeO_2} + \underset{\text{(Water)}}{2\,H_2O}$$

Where the protective magnetite film is dissolved, the parent tube metal is exposed and is susceptible to corrosion, as shown in the following Equations 2 and 3.

$$\text{Equation 2} \quad 3\,Fe + 4\,H_2O \rightarrow Fe_3O_4 + 4\,H_2$$

$$\text{Equation 3} \quad Fe + 2\,NaOH \rightarrow Na_2FeO_2 + H_2$$

The concentration of boiler water chemicals at boiler surfaces, and the ensuing corrosion, are the results of two prime mechanisms, i.e., deposit formation and film boiling, which are discussed below.

Deposit formation on boiler surfaces (dirty boiler tube surfaces) is the most common cause of localized concentration of chemicals to corrosive levels. The most prevalent deposits that can cause surface-concentration of boiler chemicals are derived from iron and copper corrosion products which enter a boiler with its feedwater. Large industrial condensate return systems or extensive feedwater heating systems (as found in a utility operation) are major sources of both iron and copper impurities which can enter the boiler. These and other contaminants may enter a boiler in soluble form, and then precipitate in the vicinity of the hot boiler surfaces. The higher temperatures at a boiler's heat-exchange surfaces will precipitate contaminants whose water-solubilities decrease at higher temperatures. The precipitation of contaminants leads to deposition on the boiler surfaces. Iron compounds form porous, insulating-types of deposits that are particularly active in promoting surface-concentration of boiler chemicals. Porous, insulating-types of deposits allow boiler water to diffuse into the deposit where the water becomes trapped and boils. The boiling of deposit-entrapped water produces relatively pure steam which tends to diffuse out of the deposit, leaving behind superheated, non-boiling equilibrium solutions of caustic. Boiler water containing, for instance, 100 ppm of of NaOH can form solutions having from about 50,000 to about 400,000 ppm NaOH (5 - 40%) upon diffusion into porous, insulating types of surface deposits.

Hydrogen cracking (embrittlement) of boiler steel can occur as an additional consequence of high temperature zone deposit accumulations, (normally found only above 1800 psig). This kind of boiler tube deterioration may accompany caustic gouging. In hydrogen cracking, atomic hydrogen formed as a result of corrosion of the tube surface (from alkali or acid attack) migrates or diffuses into the tube metal where it combines with the carbon contained in the cementite (FeC) to form methane gas, as shown in Equation 4.

$$\text{Equation 4} \quad \underset{\text{(Hydrogen)}}{4H} + \underset{\text{(Carbon)}}{C} \longrightarrow \underset{\text{(Methane)}}{CH_4}$$

Discontinuous, intergranular cracks are formed along the grain boundaries due to gas pressure buildup.

Film boiling, the second primary cause of caustic gouging, occurs when the heat input (heat flux) to a given section of boiler tube surface is so high that an orderly transfer of the heat from the tube surface to the steam-water mixture does not take place, leading to the formation of highly concentrated, nonboiling liquid films. Film boiling (which is also called "departure from nucleate boiling" or "DNB", steam blanketing, or steam disengagement) in most instances arises because the affected surface was not intended to receive direct heat input, or the surface orientation (sloped, horizontal, and so forth) is such that inadequate free rinsing occurs even though the heat inputs experienced are normal. Insufficient water flow in a tube due to design or operational considerations may also cause film boiling. Film boiling on the water-side metal surface causes an evaporative concentration of salts.

Film boiling and the problems associated therewith are generally seen with increasing frequency when:

(a) boiler pressures are increased;
(b) design heat transfer rates exceed 150,000 Btu/hr/sq ft.;
(c) boiler water circulation is disturbed due to weld backing rings, tube dents, or unusual tube or boiler designs;

and/or

(d) fireside abnormalities occur, such as unusual slagging (or deslagging) problems, or physical damage or destruction to baffles, and so forth.

The elimination or reduction of surface concentration of caustic and the resulting corrosion requires a boiler water treatment program that minimizes or excludes free hydroxide alkalinity (caustic). Low-alkalinity boiler water treatment programs exemplified by the pH/phosphate approach have become necessary. Two phosphate-based programs of the pH/phosphate type provide low-alkalinity boiler water treatments. The "TRI-AD" (or "Precision Control") program favors the maintenance of a low, tightly controlled excess of caustic. The "Congruent Control" program operates well within a "captive alkalinity zone" (described below) and essentially eliminates the potential for any free caustic. While any program that advocates alkalinity (hydrate) control by using various phosphate or phosphate-caustic ratios to maintain a safe boiler water environment may be classified being of the pH/phosphate type, only these two specific variations (TRI-AD and Congruent Control) will be discussed here in detail. Other pH/phosphate control programs and other pH/phosphate programs are within the scope of the present application, that is, they can also be controlled by the present method.

The intricate phosphate chemistry that controls these two programs requires very pure feedwater, with make-up water of demineralized or evaporated quality. Even small amounts of contamination can make control of a pH/phosphate program extremely difficult. Return condensate should be very high quality, preferably having been polished through powdered resin or deep bed ion exchange units. Table 1 below sets forth the feedwater, boiler water, and steam quality control guidelines for these programs.

Table 1

| Typical pH/Phosphate Operating Control Guidelines | |
|---|---|
| Water System/Parameter | Operating Range or Maximum (max.) |
| Returned Condensate/Feedwater | |
| Specific Conductivity | < 5 micro-mhos |
| Cationic Conductivity | < 10 micro-mhos |
| pH | 8.8 - 9.6 (see below) |
| $Na^+$ | < 5 ppb |
| $O_2$ | < 7 ppb |
| Turbidity | < 10 ppb |
| $SiO_2$ | < 5 ppb |
| Fe | 10 ppb max. |
| Cu | 10 ppb max. |
| Steam Generator | |
| Specific Conductivity | < 2000 |
| pH | 9.1 - 10.2 |
| $PO_4$ | 2 - 25 ppm |
| $SiO_2$ | (limit varies with pressure) |
| Saturated Steam | |
| Cation Conductivity | 0.1 micro-mhos max. |
| Na | 3 - 10 ppb max. |
| $SiO_2$ | 10 ppb max. |

Boiler systems containing copper alloys should have a condensate/feedwater pH within the range of 8.8 - 9.2. All-steel boiler systems are best protected when the condensate/feedwater pH is within the 9.2 - 9.6 range. A compromise condensate/feedwater pH control range of 8.8 to 9.2 is usually established where both metals are present.

The basis of phosphate-pH control is that sodium phosphates are pH buffers and the disodium phosphate specie transforms a potentially corrosive caustic-rich solution into a relatively harmless trisodium phosphate solution, as shown by the following Equation 5.

$$\text{Equation 5} \quad Na_2HPO_4 \; (\text{Disodium Phosphate}) + NaOH \; (\text{Caustic}) \longleftrightarrow Na_3PO_4 \; (\text{Trisodium Phosphate}) + H_2O \; (\text{Water})$$

While the reaction shown in Equation 5 describes the basic concept phosphate-pH control, the underlying chemistry is more complex. Many phosphates can be used in various combinations to achieve the desired result. As shown below in Equation 6, the mono-, di- and trisodium phosphates (orthophosphates) are all derived from the reaction of phosphoric acid with caustic.

$$\text{Equation 6} \quad H_3PO_4 + x\,NaOH \leftrightarrow Na_xH_{3-x}PO_4 + H_2O$$

wherein x is 1, 2 or 3. The addition of phosphoric acid or any one of the orthophosphates to water produces a hydrolysis reaction that yields phosphate ions and hydrogen ions, sodium ions, hydroxide ions or combinations, while caustic hydrolyzes to sodium and hydroxide ions, as shown in Equations 7 to 11 of Table 2 below.

Table 2

| Equations 7 to 11 | | | | | | | | | | | | | |
|---|---|---|---|---|---|---|---|---|---|---|---|---|---|
| Eq. | Chemical | | water | | $HPO_4^{-2}$ | | $H^+$ | | $H_2O$ | | $Na^+$ | | $OH^-$ |
| 7 | $H_3PO_4$ | + | $H_2O$ | ↔ | $HPO_4^{-2}$ | + | $2H^+$ | + | -- | + | -- | + | -- |
| 8 | $NaH_2PO_4$ | + | $H_2O$ | ↔ | $HPO_4^{-2}$ | + | $H^+$ | + | $H_2O$ | + | $Na^+$ | + | -- |
| 9 | $Na_2HPO_4$ | + | $H_2O$ | ↔ | $HPO_4^{-2}$ | + | -- | + | $H_2O$ | + | $2\,Na^+$ | + | -- |
| 10 | $Na_3PO_4$ | + | $H_2O$ | ↔ | $HPO_4^{-2}$ | + | -- | + | -- | + | $3\,Na^+$ | + | $OH^-$ |
| 11 | NaOH | + | $H_2O$ | ↔ | -- | + | -- | + | $H_2O$ | + | $Na^+$ | + | $OH^-$ |

Only the dibasic hydrogen phosphate ion, $HPO_4^{-2}$, is shown in Equations 7-10, because within a pH range of from about 9 to about 10.5 the distribution of the different ionic species of phosphate is at most 1 or 2 percent of the mono- or tribasic phosphate ions, and thus substantially all phosphates that are added to water within that pH range will hydrolyze to the dibasic ion.

From Equation 5 taken together with Equations 8 to 10, the following conclusions may be derived concerning the addition of sodium orthophosphates within the boiler pH range:

(1) Tribasic sodium phosphate hydrolysis releases sodium hydroxide (Equation 10). Thus, one mole of trisodium phosphate will increase boiler water pH equivalent to the addition of one mole sodium hydroxide.
(2) Dibasic sodium phosphate hydrolysis has little or no effect on pH (Equation 9).
(3) Monobasic sodium phosphate dissociates to the dibasic form; thus, a one mole addition of monosodium phosphate is capable of neutralizing one mole of sodium hydroxide or one mole of trisodium phosphate.

The solution (boiler water) pH that will result from the addition of the various orthophosphates therefore can be predicted, and graphs showing the phosphate-pH relationship in terms of phosphate concentration as $PO_4$ (in ppm) versus pH of an aqueous solution for various $Na:PO_4$ ratios are available in boiler literature. (Since orthophosphates are comprised of sodium and phosphate in molar ratios of 1, 2 and 3, it is common practice to describe an orthophosphate or a mixture of orthophosphates in terms of its sodium-to-phosphate molar ratio, $Na:PO_4$).

Sodium hydroxide generated solely by the hydrolysis of trisodium phosphate (Equation 10) is sometimes called "captive" because it will revert to trisodium phosphate at any site of localized evaporative concentration. Such reversion of sodium hydroxide to trisodium phosphate in confined areas such as within and/or under surface deposits avoids the formation of pockets of concentrated sodium hydroxide and thus prevents caustic-gouging type of metal attack. In Equation 10, when water is removed upon evaporation, the equilibrium is forced to the left. A complete evaporation to dryness would leave a residue of trisodium phosphate free of sodium hydroxide, but incomplete evaporation is the more likely condition beneath a porous deposit. Incomplete evaporation produces a liquid underneath the deposit that is rich in sodium hydroxide, particularly if incipient localized corrosion is already occurring. Thus the maintenance of a 3:1 sodium phosphate ratio in boiler water may not provide positive protection against caustic-concentration-type

corrosion damage. One form of pH/phosphate control maintains a $Na:PO_4$ ratio that does not exceed 2.6. (A $Na:PO_4$ ratio of 2.6 corresponds to a 3:2 blend of trisodium and disodium phosphate.) The differing hydrolysis effects of different sodium phosphates when selectively adjusting pH, $PO_4$, or both, to keep pH and $PO_4$ coordinates within the desired range are shown in a control diagram discussed below.

The primary objective of a pH/phosphate program, whether of the TRI-AD or Congruent Control type, is controlling the presence or absence of free sodium hydroxide. At a 3.0:1 $Na:PO_4$ mole ratio or greater, only the equivalent of trisodium phosphate plus caustic exists. At $Na:PO_4$ mole ratios below 3.0:1 (down to a 2.0:1 mole ratio), a mixture of disodium and trisodium phosphate is present. In Figure 3 is shown a pH/phosphate control curve which includes the equilibrium boundary ("ratio 3.0" line) between the trisodium phosphate/caustic combination and the trisodium/disodium combination for pH values from about 8.2 to about 10.6, which forms the basis for all phosphate-pH programs. The line represents the points (combinations of pH values versus phosphate concentrations) at which there exists a 3.0:1 $Na:PO_4$ mole ratio and only the trisodium phosphate specie is present (the phosphate-pH relationship for trisodium phosphate or 3.0:1 $Na:PO_4$ line). Above that boundary, the equivalent of trisodium phosphate and caustic exist. Below that boundary, a mixture of tri- and disodium phosphate exists.

The TRI-AD pH/phosphate program operates above the 3.0:1 $Na:PO_4$ line (Figure 3); the Congruent Control pH/phosphate program operates below the 3.0:1 line, which is referred to in the technical field as the "captive alkalinity" zone.

As boiler water of a given $Na:PO_4$ ratio is concentrated at local areas of the boiler (under deposits or in zones of film boiling), the sparingly soluble natures of the various phosphate species at boiler water pH values and temperatures cause precipitation (solid phase formation) to occur. The solid phase $Na:PO_4$ ratio will always be less than 3.0:1, but the actual solid phase stoichiometry will vary with the existing liquid phase $Na:PO_4$ ratio. When the liquid phase $Na:PO_4$ ratio is greater than 2.85:1, the solid phase formed upon precipitation will have a lower $Na:PO_4$ ratio than the liquid phase. Phosphate precipitation when the liquid phase $Na:PO_4$ ratio is greater than 2.85:1 therefore results in an increase in the liquid phase $Na:PO_4$ ratio and causes a shift towards the 3.0:1 $Na:PO_4$ ratio line, above which free sodium hydroxide will exist, creating potential corrosion problems. Conversely, if the liquid phase $Na:PO_4$ ratio is less than 2.85:1, the solid phase Na:PO4 ratio is always greater than the liquid phase ratio. Phosphate precipitation when the liquid phase $Na:PO_4$ ratio is less than 2.85:1 therefore depletes the water system of sodium, decreasing the $Na:PO_4$ ratio of the remaining liquid phase. Precipitation thereby moves the water system even further below the 3:1 line, rather than toward it.

A plot of the composition of solid in terms of the $Na:PO_4$ ratio versus the composition of solution phase solid in terms of the $Na:PO_4$ ratio in an equilibrium system of disodium phosphate/water at a given temperature will cross a congruence line (line formed of points representing equal solid and solution $Na:PO_4$ ratios) at what is known as a point of congruency, or simply the congruent point. For instance, at a temperature of 572 °F (300 °C) the congruent point is where both the solution phase and the solid phase have an identical $Na:PO_4$ of 2.85:1. At 689 $^0$F (365 °C), the congruent point is about 2.6:1. Since these congruent points form a line of demarcation between the zones where you are either moving towards or away from the 3:1 line under conditions of precipitation, these points become the upper control boundary for the Congruent Control method. Typically under Congruent Control the phosphate-pH relationship is controlled to maintain a solution phase (boiler water) $Na:PO_4$ ratio range of 2.6:1 to 2.3:1. The 2.3:1 ratio is chosen as the bottom of the control range to avoid the formation of acid phosphate which can occur below 2.13:1.

"Phosphate hideout" and "chemical hideout" are terms used to describe the loss of boiler water phosphate residual that accompanies this solid phase formation problem. Typically, phosphate tends to "disappear" as load (steaming rate load) is increased towards maximum. The pH will also vary, usually in an upward direction, although one would expect that the change in pH would be consistent with the change in $Na:PO_4$ ratio of the solution. The upward trend in pH may be explained by an interaction between phosphate and magnetite that is also known to exist. Just as phosphate disappears with increasing load, so does it reappear with decreasing load, with concurrent impact on the system pH. Dissolution of the solid phase occurs as load is reduced.

The phosphate hideout phenomenon can pose a significant chemistry control problem in high pressure boilers that experience load variations. In some severe instances "all-volatile treatment" (AVT) programs, for instance using only nitrogen-hydrogen compounds such as ammonia and hydrazine, have been adopted to avoid the control problems caused by phosphate hideout. AVT programs, however, are not universally appropriate. In an All Volatile Treatment program the boiler water is unbuffered and thus it is subject to extensive and rapid pH excursions in the event of feed water contamination, and therefore such a program has no tolerance for condenser coolant inleakage unless condensate polishing is provided. An AVT program Is therefore a realistic alternative only when feedwater and condensate quality meet the rigorous standards required for AVT.

In the 1000-1800 psi (70,307-126,553 grams/sq. cm) range (large industrial boilers and small utility boilers), a TRI-AD program that incorporates the maintenance of very low levels of free hydroxide alkalinity have at times been preferred over the more stringent Congruent Control approach. The TRI-AD program control, however, is not less precise, and the control principles are not any less complicated, than a Congruent Control program. (In fact, as shown in Figure

3 discussed further below, the boiler water total solids level on a TRI-AD program may actually be lower than on Congruent Control program.) A TRI-AD program's primary advantage in systems where it has been shown to be acceptable is that it provides a small reservoir of excess caustic that can:

(a) help to condition any hardness sludge that may form as the result of feedwater contamination; and
(b) buffer against unusual downward excursions in pH, which also typically are a result of feedwater contamination, particularly when the feedwater contamination is due to a condenser leak.

A TRI-AD program cannot be used when operating experience indicates a system intolerance to free caustic, nor when the pressure is above 1800 psi (126,553 grams/sq. cm).

In Figure 3 are shown the control parameters for coordinated programs of the Congruent Control and TRI-AD types. Figure 3 is comprised of plots of the total orthophosphate in the boiler water, in terms of ppm $PO_4$ versus boiler water pH at 25 °C (77 °F). Figure 3 also shows the specific phosphate - pH coordinate boundaries based on boiler drum pressure. Shown is a Box A (circumscribing Area A) and a Box B, Box B encompassing the combined Area A and Area B. Also shown in Figure 3 is Box C (circumscribing Area C) and Box D, Box D encompassing the combined Area C and Area D. Both of Boxes C and D are bounded by the 2.3 and 2.6 $Na:PO_4$ ratio lines. Using a TRI-AD program (Box A and Box B), a boiler operating in the 1000-1500 psi range could use $PO_4$ - pH coordinates over the combined A and B areas, whereas a boiler operating in the more critical 1500-1800 psi range is restricted to Area A. The Congruent Control program, which as mentioned above is a pH/phosphate - pH program that takes its name from the concept of a point of congruency existing between solid phase and liquid phase $Na:PO_4$ ratio, seeks to minimize, if not eliminate, the possibility of developing free caustic anywhere in the boiler. It operates in Boxes C and D, between the 2.6 and 2.3 $Na:PO_4$ ratio lines. Any phosphate hideout (*e.g.*, precipitation due to localized areas of evaporation, hence potential corrosion) results in a swing in the $Na:PO_4$ ratio away from the 3:1 line, and thus away from potential "free" hydroxide alkalinity.

Most modern utility boilers use the Congruent Control mode of the pH/phosphate-pH concept. A complete program routinely further includes hydrazine or another oxygen scavenger, and a neutralizing amine or ammonia for feedwater and condensate pH control.

The control vectors on Figure 3 provide the direction in correcting a transient (fugitive) condition in control. A line drawn between the current, or existing, $PO_4$/pH coordinates versus the desired $PO_4$/pH coordinates identifies the appropriate directional vector that indicates which phosphate (or phosphates) need to be added, or whether caustic addition or boiler blowdown may be more appropriate. These vectors are founded on the basic orthophosphates hydrolysis reactions discussed above.

In either the TRI-AD or the Congruent Control program, actual choice of phosphate product(s) (phosphate feed) used to control the system may vary with plant or company preference. Liquid phosphate products are considered by some plants the most convenient form and these are commercially available, for instance under the trade names of Nalco 7208 and 7209 from Nalco Chemical Company of Naperville, Illinois. Phosphate feeds comprised of monosodium phosphate and caustic in combination may provide wide flexibility. Mixtures of two or more phosphates, changing the ratio as needed to maintain control, are used in some plants. In some cases, there may be sufficient "outside" sodium alkalinity entering the system (most often as a result of sodium alkalinity inleakage) to allow standardization on one phosphate feed source, but typically the outside sodium alkalinity level varies significantly and thus instead the phosphate feed mixture requires constant adjustment when outside alkalinity is entering the boiler.

The conventional servicing and testing of high pressure boiler systems is conceptually no different than for lower pressure boiler systems, and most of the same test procedures are used, although the lower concentration levels of a conventional pH/phosphate program control require exemplary analytical technique. In most plants, control of boiler water treatment will be under the direction of a chemist. A water treatment laboratory may be only minimally equipped or may have an array of sophisticated equipment (atomic adsorption, specific ion electrodes, spectrophotometers, and the like). In addition to laboratory analysis capability, most plants will also have several continuous reading automatic analyzers throughout the plant to support the maintenance of the stringent water quality requirements of pH/phosphate programs.

## Phosphate Concentration

The present invention includes the determination of the phosphate concentration in the feedwater and optionally determining whether there is a difference between the actual feedwater concentration of the phosphate and the target feedwater concentration and correcting the dosage of the phosphate if a difference is detected. In these steps, at least one phosphate is added to the fresh feedwater prior to charging it to the boiler to provide a target concentration of the phosphate in the boiler water. The additional steps are:

1. Adding a tracer to the fresh feedwater in a dosage of known proportion to the phosphate and/or phosphate-containing treatment product being added to the feedwater;
2. Sensing a characteristic of the tracer in the feedwater downstream of its addition point that is equivalent to its concentration in the feedwater;
3. Converting the sensed characteristic to a value equivalent to the concentration of the inert tracer in the boiler water;
4. Determining from the value equivalent to the concentration of the tracer a value equivalent to the concentration of the phosphate in the feedwater, and optionally whether a difference between the target concentration and the actual concentration of phosphate exists; and
5. Correcting the dosage of phosphate if the determination shows the existence of such a difference.

For the determination of phosphate concentration, the tracer may be inert or active, as discussed below, and the tracer characteristic sensing is conducted at a point that is preferably downstream of the tracer and phosphate addition point(s) and upstream of point that the feedwater enters the boiler compartment.

Boiler Cycles

A boiler's cycles of concentration is an operating parameter that should be well monitored. Limits on boiler cycles of concentration, so as to limit the maximum impurity concentration within a boiler, are routinely set by boiler and turbine manufacturers, water treatment companies and the industrial plants employing the boilers. Such limitations are intended for, and are generally necessary to, the avoidance of serious scale formation/deposition on internal boiler surfaces and/or boiler-related equipment surfaces within a boiler, despite an otherwise adequate water treatment program. Boiler feedwater, which normally is comprised of both makeup water and recirculated condensate water, contains some impurities regardless of the extent to which such waters are treated before being fed to a boiler. When steam is generated, substantially pure $H_2O$ vapor is discharged from the boiler, leaving the impurities (the dissolved and suspended solids) behind, which increases their concentration in the boiler water. The discharged steam is replaced by contaminant-containing feedwater. An ever increasing concentration of dissolved and suspended solids in the boiler water would inevitably result in very serious problems, including deposit formation, corrosion, foaming, mechanical and selective carryover, decreased heat transfer efficiency, boiler tube failure or occlusion, and the like. Boiler-impurities concentration (boiler solids concentration) is offset by withdrawing water as blowdown. The heat energy in the blowdown, however, is a major factor reducing a boiler's thermal efficiency, and therefore a blowdown rate in excess of that required to limit the solids concentration is preferably avoided. An excessive blowdown rate also unnecessarily increases water costs.

The ratio of solids concentration in the blowdown to the solids concentration in the feedwater is called the "cycle value", "cycles of concentration", "concentration cycles" or "cycles" of a boiler operation. If a boiler is being operated on too low of a concentration cycle, the blowdown rate is too high, heat energy is lost and the boiler operation is unduly inefficient. The needless loss of heat with unrequired blowdown decreases thermal efficiency, and water costs increase at the same time. If a boiler is being operated on too high of a concentration cycle, the blowdown rate is too low and the potential for solids-derived problems, such as scale deposition and corrosion (discussed above), are increased. Thus the cycles of concentration is one of the primary operating parameters of a boiler.

The method of the present invention includes the monitoring of a boiler operation's cycles of concentration. U.S. Patent No. 5,041,386, Claudia C. Pierce, Roger W. Fowee, and John E. Hoots, issued August 20, 1991, incorporated hereinto by reference, discloses the use of inert tracers to monitor boiler concentration cycles. Boiler cycles are calculated by adding an inert tracer to the feedwater being charged to the boiler in a known concentration, and then determining an analog of its concentration in the blowdown. If the cycles value does not compare to the standard operating value, which may be the cycles value proposed by the boiler manufacturer or set by the operator or suggested by the water-treatment supplier, then the blowdown rate and/or the dosage of water-treatment agent can be adjusted. The inert tracer preferably is employed to determine the cycles (impurity or contaminant concentration within the boiler water) on a continuous basis. The inert tracer may be used for other purposes, such as the determination of percent holding time (half-life time), as a reference standard in the monitoring of concentration of water-treatment agent concentration level, and the like, concomitantly with the cycles determination.

Industrial boilers often have heat transfer rates in excess of 100,000 $Btu/ft^2$-hr (2,500 $cal/m^2$-hr) and the presence of even an extremely thin deposit layer within the boiler could cause a serious elevation in the temperature of the tube metal. Therefore, as discussed above, the required feedwater purity is very high and the permitted concentration of impurities introduced with the feedwater is very low. These are often high cycles value boilers with almost constant steam generation demands.

Boiler cycles (cycles value, concentration cycles, cycles of concentration and the like) as such terminology is used herein, as understood generally in the boiler field, is the ratio of the average concentration of a particular impurity or

component ("IMP") in the blowdown at steady state (average final concentration or "$C_F$") to its concentration in the feedwater (initial concentration or "$C_I$"), which ratio is determined from the following Formula I:

$$\text{Formula I} \qquad \text{cycles} = (C_F)/(C_I) \text{ or } \frac{\text{steady state blowdown concentratrion of IMP}}{\text{feedwater concentration of IMP}}$$

and the value, which is an equilibrium value, will always be greater than one since the impurity IMP in the blowdown is always more concentrated than in the feedwater due to water removal as steam. (Upsets in feedwater quality of a magnitude sufficient to lower the cycles value to less than one are not tolerable in the boiler field and would never occur outside of a major boiler operational failure.)

In the process of the present invention, the inert tracer is the "impurity" or "IMP" of Formula I above, and the acronym "IT" for "inert tracer" can be employed in substitution for the "IMP" designation. When the cycles values of intermediate and high pressure boilers are monitored with inert tracers (for instance those disclosed in U.S. Patent No. 5,041,386) that do not appreciably carry over into the steam, and which can be selectively detected at very low concentrations (for instance 0.005 ppm or less), it has been found that in a given boiler system not only can the average cycles value be determined from the average $C_F$, but the normal range of the $C_F$ fluctuation can also be determined.

The concentration of the inert component in the boiler at steady state varies from a high concentration "$C_H$", having a value that is higher than $C_F$ (the value of the average concentration of an inert component in the blowdown), to a low concentration "$C_L$", having a value between the ($C_I$) and the ($C_F$), within a time period A as shown graphically in Figure 2. The high concentration $C_H$ of the inert tracer in a boiler for a uniformly cycling boiler system is of course higher than the average $C_F$ of the blowdown. The low concentration $C_L$ of the inert tracer in the boiler is lower than the average $C_F$ of the blowdown but, since the boiler water is never entirely replaced with feedwater during cycling, it is never as low as $C_I$.

The present invention contemplates the phenomenon that in any practical boiler system operation the $C_F$ is not an absolute constant and the cycles value assigned to a boiler operation is based on an average value of $C_F$. The monitoring of a boiler cycles value with an inert tracer can also be done for boilers that are not intermediate or high pressure boilers. (The only boiler operation parameters that may exclude the use of some of the preferred embodiments of present invention are pressures in excess of 1,800 psig, which pressures may lead to decomposition of the presently preferred sulfonated naphthalene inert tracers that are described below.)

Steady states as such terminology is used herein and as understood generally in the boiler field, is the condition that exists when the concentration of the inert tracer (or other stable substance introduced into the boiler with the feedwater) in the water of the boiler system reaches a uniform, repetitious cycling fluctuation of the inert tracer from a predictable high concentration ($C_H$) to a predictable low concentration ($C_L$), which state is reached when the transient conditions arising at the start of the inert tracer feed to the feedwater have become negligible. For purposes of the present invention, a boiler can be considered operating at a steady state with respect to the inert tracer when there is no significant change in the fluctuation of the inert tracer between $C_H$ and $C_L$ of the concentration of the inert tracer in the boiler and no significant change in the blowdown rate, the feedwater rate, the rate of feeding the tracer to the boiler (or concentration of tracer in the feedwater when introduced therewith) and steaming rate (in the absence of boiler leakage). To detect a boiler's cycles value employing the present process, a steady state as to inert tracer concentration must first be reached, and preferably the $C_L$ and/or $C_H$ values or values proportionate thereto, determined.

As mentioned above, the cycles-determination step of the present process can be activated only after a steady state as to the inert tracer has been reached. The time required for reaching such steady state after a uniform dosage (feed rate) of inert tracer has begun can be calculated from the following Formula II:

$$\text{Formula II} \qquad t = -(M/B) \ln (1 - C_T/C_F)$$

wherein M is the mass of the boiler water (in lb.), B is the blowdown rate (in lbs/hour), $C_F$ is the concentration or average concentration of the inert tracer in the blowdown after steady state is reached, and $C_T$ is the concentration of the inert tracer in the blowdown at time t. The M/B factor is a constant (boiler constant "K") for a given boiler operating under a uniform blowdown rate. M can be determined from design documents or by spiking the boiler quickly with a known amount of an inert tracer and measuring the value $C_F$ obtained with the blowdown shut off. If the mass of the boiler water is unable to be known precisely, a determination that a steady state as to the inert tracer has been reached is made when a uniform average cycles value is seen by monitoring the inert tracer for a period of about one to two weeks while holding $C_I$ constant.

The cycles determination of the present method is comprised of the following steps:

1. Employing as the inert component an inert tracer added to the feedwater in a known concentration ($C_I$);

2. Sensing a characteristic of the inert tracer in the boiler at steady state equivalent to its concentration in the boiler water;
3. Converting the sensed characteristic to a value equivalent to the concentration of the inert tracer in the boiler water;
4. Optionally recording the cyclic concentration fluctuation from $C_H$ to $C_L$ of the inert tracer in the boiler water;
5. Determining the cycles value of the boiler from the equation

$$\text{Formula III} \qquad \text{cycles} = (C_F)/(C_I) \text{ or } \frac{\text{steady state blowdown concentration of IT}}{\text{feedwater concentration of IT}}$$

6. Optionally controlling the blowdown rate based on the above determination of the cycles value.

The present invention may be employed regardless of a boiler's normal blowdown flow operation and control. In many large boilers the blowdown valve is normally open and the blowdown rate would not normally fall to zero. Instead the blowdown valve adjusts the blowdown rate depending upon some indicated impurity build-up within the boiler, which indication may be based on concentration readings of an inert tracer in the boiler as it fluctuates between $C_H$ and $C_L$. A balance between the rate feedwater is flowing into the boiler and the rate steam is being discharged is generally seen in this type of boiler.

A pH/phosphate program can also be controlled in a boiler with open/close blowdown valve operations which has a valve frequency time period B measured from the closing of the blowdown valve, shutting off the blowdown flow, to the time at which the blowdown valve would normally reopen. In such a boiler the time period B would be about equivalent in length to time period A.

In preferred embodiments of the present invention, the cycles value determination is performed by monitoring an inert tracer in both the feedwater and in blowdown from the boiler and the phosphate feedwater concentration determination is performed by monitoring an active or inert tracer in the feedwater. At the time of its discharge, any blowdown stream will have the same composition as the water retained within the boiler system at that time. The concentration of any substance in blowdown, therefore, is commensurate with the concentration of that substance in the boiler at the time the blowdown is discharged from the boiler. Blowdown, as such terminology is understood generally in the boiler field, is water discharged in some manner from a boiler system. A sidestream that continuously taps the boiler water for a continuous monitoring of tracer concentration within the boiler would be considered a continuous blowdown stream under this general definition. The concentration of the inert tracer in the boiler at any point in time is preferably determined from the concentration of the tracer in a sidestream that taps the boiler water, which preferably is a sidestream off the blowdown line and which preferably taps the boiler water on a continuous basis. The stream of water from a boiler that is monitored for purposes of the present invention may be, for instance, a sidestream off a constantly-flowing blowdown stream. Such monitored boiler-water stream also may be a stream from a separate boiler outlet, or a sidestream from the blowdown line ahead of the blowdown valve in a boiler having an intermittent blowdown flow. In any of such instances the monitored stream is not only a part of the blowdown, but also of a generally known rate.

The inert tracer selection(s) for the purposes of the present invention must consider the temperature constraints existing on the waterside of a boiler. The inert tracer preferably is a fluorescence tracer, as discussed below.

<u>Determination of Boiler Cycles</u>

In FIGURE 1 there is shown in diagrammatic form a boiler system designated by the general reference numeral 10, comprised of a boiler 12, a feedwater line 14 through which feedwater flows into the boiler 12, a steam line 16 through which the steam generated leaves the boiler 12, and a blowdown line 18 through which blowdown is discharged from the boiler 12. The delivery of feedwater to the boiler 12 is controlled by a feedwater pump 19. The discharge of blowdown from the boiler 12 is controlled by a blowdown valve 20. A sidestream 28 off the blowdown line 18 supplies a small continuously cooled sample stream for monitoring the concentration of the inert tracer in the boiler by means of appropriate instrumentation 30, which is shown diagrammatically in FIGURE 1, and is discussed in more detail below. A feedwater sidestream 32 is tapped off of the feedwater line 14 and along such feedwater sidestream 32 is a feedwater-monitoring instrumentation 34, discussed below. The rate at which blowdown is discharged from the boiler 12 is dictated by the balance desired between the rate of introduction of impurities ("solids") to the boiler 12 together with feedwater and the rate of solids discharge from the boiler 12 with blowdown. The desired balance is normally an equal balance. The solids discharge rate should equal the solids introduction rate over a given time period. The concentration of solids within the boiler 12 at any given time, after steady state is reached, should also fall within predetermined limits.

This balance between the rate of introduction of impurities ("solids") to a boiler together with feedwater and the rate of solids discharge from the boiler with blowdown may be represented by a hypothetical boiler operation example

as follows:

(1) The maximum solids concentration within the boiler at any given time is set for an equivalent of 10 mg solids per liter of boiler water;
(2) The feedwater has an average solids concentration of 1 mg/liter, is fed to the boiler at a rate of 1,000,000 lb/day and thus 1 lb. of solids are fed to the boiler per day;
(3) The average solids concentration in the blowdown is equal to the 10 mg/liter solids concentration maximum (see 1 above), 1 lb. of solids must be discharged per day (see 2 above) and thus the blowdown rate must be 100,000/lb per day; and
(4) Steam having an essentially zero solids content is generated and discharged from the boiler at a rate of 900,000 lb. per day.

The desired cycles value for such a boiler balance is 100/10 = 10. That is, the concentration of solids in the blowdown should be 10 times the concentration of solids in the feedwater. However, the typical feedwater solids concentration, due to the pretreatment system operation and the quantities of returned condensate, will have between a 5% and 25% variance range. The more pure the feedwater, the greater the percentage variance range and thus the more difficult it becomes to make accurate boiler cycle measurements from normal feedwater impurities. The actual cycles value is more accurately measured by continuously adding a uniform dosage of an inert tracer to the feedwater and monitoring its concentration in both the feedwater and blowdown, along the feedwater line and blowdown line respectively, and substituting the concentrations determined into Formula I. (It is desirable but not required that the inert tracer be monitored along the feedwater line for the cycles determination portion of the present invention, but a tracer, which may or may not be inert, must be monitored along the feedwater line for the phosphate concentration determination portion of the present invention. Moreover, while using the same tracer for dual feedwater/blowdown cycles analyses is convenient, separate tracers can of course be employed.) If the cycles value determined by the inert tracer is lower than desired, the blowdown rate can be lowered, and if the cycles value determined by the inert tracer is higher than desired, the blowdown rate can be increased.

When the blowdown rate is set to provide the desired cycles value, the sidestream 28 off the blowdown line 18 (which need only comprise a negligible fraction of the boiler water) is monitored to determine on a continuous basis the concentration of the inert tracer(s) in the boiler water. The sidestream 28 is shown ahead of the blowdown valve 20, which is a preferred position for the sidestream 28 regardless of the normal blowdown valve operation. The concentration of the inert tracer added in known proportion to the feedwater, as monitored by the instrumentation 30, will normally fluctuate from the $C_L$ low value to the high value $C_H$ as described above. The instrumentation 34 may include one or more setpoints to support the continuous monitoring of the boiler cycles. A low setpoint, for instance, may be at or somewhat below the normal $C_L$ value. A high setpoint may be at or somewhat below about $C_H$. For instance, if the inert tracer concentration in the feedwater of a boiler having a cycles value of 50 is 1 ppm, its average concentration in blowdown (average $C_F$) would be 50 ppm. Its high value in the boiler $C_H$ may be about 55 ppm, and its low value $C_L$ may be about 45 ppm. If the tracer concentration in the boiler fell to 40 ppm, there would be a 20 percent difference between this reading and the average $C_F$ reading of 50 ppm, and a readily detectable difference of about 11 percent between this reading and the normal low inert tracer reading $C_L$. There may also be a change in the feedwater rate if the boiler is a constant steam load boiler, but this feedwater rate change would be extremely difficult to detect with any assurance. A normal feedwater rate analyzer has an accuracy of about ± 3% and a feedwater rate change would only be on the order of only about 0.5%. (In a constant steam load boiler, the feedwater rate is the steam rate plus the blowdown rate, and the steam rate for a boiler having a cycles value of about 50 would be about 50 times the blowdown rate. Flow meters to determine blowdown flow rates often have accuracy problems because of deposition and erosion.)

The downswing in the tracer concentration to its $C_L$ value occurs when blowdown water leaves the boiler with a proportional amount of inert tracer and the feedwater control system has replaced that blowdown with water containing a lesser concentration of the inert tracer, diluting the overall tracer concentration in the boiler water. The concentration of the inert tracer is not affected by the feedwater purification efficiency, or other variable parameters of the feedwater. The water loss and replacement, and the concomitant inert tracer dilution, is detectable and measurable to a precise degree. For example, a change in blowdown rate that causes only a 0.5% increase in water consumption by the boiler would also provide a 20% decrease in the tracer concentration from its average of 50 ppm. The amount of the water consumption and the precise dilution of the inert tracer need not be quantitatively determined, although such quantitative determinations can be easily made using the method of the present invention. The fluctuation of the inert tracer concentration in the blowdown from $C_H$ to $C_L$ or vice versa during time period A can provide a graphical record of the boiler cycles as shown in FIGURE 2.

The present invention of course goes farther than just the determination of the cycles value. For purposes of the present invention the same inert tracer or a another tracer is introduced into the boiler system in a known and uniform proportion to the phosphate, and preferably, but not necessarily, it is introduced into the boiler system as a component

of the phosphate feed to the boiler or boiler feedwater in a known and constant concentration proportion with respect to the phosphate. Since routinely the phosphate feed rate will be in constant proportion to the feedwater feed rate when the cycles value of the boiler is accurately determined and set at its optimal value, at least over a time period of several days or a week or so, a separate (another) tracer to measure the phosphate feed can, but need not, be used, and instead a single inert tracer may be used for both the phosphate concentration and boiler cycles determinations. The process of the present invention in an embodiment preferably includes a continuous monitoring of the concentration of a single inert tracer in the feedwater and in the boiler blowdown for both the phosphate concentration and boiler cycles determinations.

In the process of the present invention the boiler cycles and phosphate concentration are determined, and a pH/phosphate program is controlled through an enhanced technique. As shown in Figure 3 for the TRI-AD and Congruent Control types of pH/phosphate programs (although the present invention is not limited to these types of pH/phosphate programs), a line drawn between the existing $PO_4$/pH coordinates and the desired $PO_4$/pH coordinates identifies the appropriate directional vector that indicates what parameter needs to be increased to approach or regain the target coordinates. For instance, with reference to Figure 3, if the line drawn is directed to about a 7 o'clock position, it substantially coincides in direction with the blowdown control vector, and increasing the blowdown rate will take the system towards the desired $PO_4$/pH balance (corresponding to the desired coordinates on the graphs). If the line drawn is directed to about a 3 o'clock position, it substantially coincides with the disodium phosphate control vector, and increasing the disodium phosphate concentration (feed rate) will take the system towards the desired $PO_4$/pH balance. If the line drawn is directed to about a 9 o'clock position, it substantially falls between the blowdown control vector and the caustic control vector, and increasing both the blowdown rate and the caustic concentration (by increasing the caustic feed rate) in needed proportion to provide in combination the correct vector as direction and length will take the system towards the desired $PO_4$/pH balance.

Referring again to Figure 1, the process of the present invention comprises:

(1) determining the phosphate concentration (feed rate) (which in preferred embodiments is commensurate with the determination of the feedwater feed rate) with a tracer using instrumentation 34 along the sidestream 32 off the feedwater line 14;
(2) determining the boiler cycles with a tracer (the same tracer or different tracers) using at least instrumentation 30 along the sidestream 28 off the blowdown line 18;
(3) determining the blowdown pH, for instance with a continuously-reading pH meter or other suitable instrumentation, shown in Figure 1 as instrumentation 36 downstream of instrumentation 30 along the sidestream 28 off the blowdown line 18;
(4) optionally determining the hydrate alkalinity concentration of the feedwater, for instance with an Orion 1000 series or other suitable sampling/analysis instrumentation, shown in Figure 1 as instrumentation 38 downstream of instrumentation 34 along the sidestream 33 off the feedwater line 14; and
(5) furnishing the data defining all of these value to a control center 40.

The control center 40, which preferably is a computer, provides a responsive control of the caustic pump 44 (controlling caustic feed), the phosphate pump 46 (controlling phosphate feed), and the blowdown valve 20 (controlling blowdown rate). The responsive adjustments of the control center 40 thus are directed to the parameters subject to potential adjustment to approach or regain the desired $PO_4$/pH balance (control box $PO_4$/pH coordinates as shown in Figure 3, or control box $PO_4$/pH coordinates of like $PO_4$/pH plots of pH/phosphate programs).

Referring to Figure 3 also, for any pH/phosphate program it may be desirable to maintain a specific Na:$PO_4$ mole ratio ("$R_m$"), for instance a $R_m$ within the range of 2.3 to 2.8 within the boiler. $R_m$ is related to the Na:$PO_4$ concentration by molecular weight ratio ("R") by the factor of 4.13 (that is, 95/23). The $R_m$ values are shown on Figure 3 as lines, some of which provide the upper or lower boundaries of the control boxes. While there is some system demand for phosphate in a pH/phosphate program, it is not a high system demand, and more important than the actual unprecipitated phosphate concentration value in the boiler 12 is that a uniform phosphate residual level is maintained within the boiler 12. The two feedwater monitorings, that is the feedwater analysis of the sodium concentration using sampling/analysis instrumentation 38 and the feedwater tracer (proportional to the phosphate being fed) using instrumentation 34 are sufficient to provide the control center 40 with values for the calculation of R and/or $R_m$, whichever is more convenient. The pH monitoring of the blowdown using instrumentation 36 provides the control center 40 with the existing pH value within the boiler. The determination of the actual boiler cycles through at least the monitoring of an inert tracer at least in the blowdown using instrumentation 30 provides the control center 40 with the existing blowdown rate. Based upon this input of values, the control center 40 will produce the modifications in the caustic feed, the phosphate feed, and/or the blowdown rate by appropriate adjustments to the caustic feed pump 44, the phosphate feed pump 46, and/or the blowdown valve 20 that will create accurate overall program vector adjustments within the control box.

The present invention provides a pH/phosphate control method that is cost efficient and is relatively simple to

operate; it reduces operator testing commitments, improves boiler efficiency and reliability, and improves the process capability of the treatment program. The present invention provides a pH/phosphate program control process of great sensitivity that is independent of fluctuations in a boiler's steaming rate or phosphate hideout tendencies. The present invention provides a pH/phosphate program control method of great sensitivity that is independent of small quality variations in boiler feedwater.

The present process also overcomes problems with phosphate "hideout", which causes operators to make incorrect program adjustments in their efforts to stay within their control parameter range. The phosphate concentration for purposes of pH/phosphate program control in the process of the present invention is monitored in the feedwater. The phosphate hideout seen as a loss of boiler water phosphate residual that accompanies a solid phase formation is a control problem only when boiler-water phosphate residuals are being measured by the phosphate concentration seen in the blowdown. The boiler-water phosphate, as measured by blowdown phosphate concentration, disappears with increasing load, and reappear with decreasing load, with concurrent impact on the system pH. The process of the present invention is independent of blowdown phosphate concentration measurements, and hence immune to erroneous control adjustment triggered by the phosphate hideout phenomenon. The process of the present invention is thus particularly advantageous for use in high pressure boilers that experience load variations, which otherwise may adopt an AVT program to avoid the control problems caused by phosphate hideout, or which are plagued by phosphate hideout control problems because their feedwater and condensate quality do not meet the rigorous standards required for AVT.

Inert And Active Tracers

The terms "tracer" or "tracer(s), as used herein, refer to any and all of the tracers used in the present process, regardless of whether a given tracer is active or inert and regardless of whether a given tracer is a "feedwater tracer", a "phosphate-feed tracer" and/or a "blowdown tracer" as these terms are used and defined herein. The term "inert tracer", as used herein, refers to only tracers that are inert, as defined below. The term "active tracer", as used herein, refers to tracers that provide some performance benefit downstream of the sample point and/or are consumed or have their tracer characteristic changed by chemical alteration to some extent within the boiler system downstream of the sample point. A feedwater tracer is any tracer monitored in the boiler feedwater upstream of the feedwater inlet to the boiler for any purpose of the present invention, and it may be an inert tracer or an active tracer. A phosphate-feed tracer is a tracer monitored in any stream containing the phosphate feeding to the boiler (which stream often but not necessarily is the feedwater stream downstream of phosphate pump) for the purpose of monitoring the phosphate feed rate. The phosphate-feed tracer may be an inert tracer or an active tracer, and it may also be the feedwater tracer. The phosphate-feed tracer of course is present in the stream in which it is monitored in known proportion to the phosphate so that its concentration can be correlated to the phosphate concentration. A blowdown tracer is a tracer monitored in the blowdown for the purpose of determining boiler cycles. The blowdown tracer must be inert, and it may also be the feedwater tracer and/or the phosphate-feed tracer. Thus a single inert tracer may optionally be the feedwater, phosphate-feed and blowdown tracer, while an active tracer can only be the feedwater and/or the phosphate-feed tracer. A single inert tracer that acts as a feedwater, phosphate feed and blowdown tracer is referred to herein as the solitary tracer.

When a tracer(s) is employed for the primary and/or sole purpose of being a feedwater and/or phosphate-feed tracer for the present process, it may or may not be an inert tracer, as discussed above. If a tracer is not inert, another, and inert, tracer(s) is also employed. In other words, at least one inert blowdown tracer must be used to monitor the boiler cycles, and such inert blowdown tracer is preferably, but not necessarily, also the feedwater tracer and/or phosphate-feed tracer.

A phosphate-feed or solitary tracer would typically be charged to a water system together or concomitantly with the phosphate treatment agent. Such tracer(s) may be a component of a formulated phosphate product, and in most instances it would be a compound(s) added to the formulated phosphate product, or otherwise added to the water system in proportion to the phosphate. After introduction to the boiler it would not precisely follow the phosphate in the system, and instead it can then act as a blowdown tracer for the boiler-cycles determination purposes of the present invention. Given the capability of an inert tracer to function as the phosphate-feed tracer, as a feedwater tracer, and as the blowdown tracer for boiler cycles determination purposes, there would often be no benefit in, or practical reason for, the use of another tracer. Nonetheless the use of two or three inert tracers separately functioning as phosphate-feed, feedwater and/or blowdown tracers is not excluded from the process of the present invention, and in some instances there may be a benefit in, or practical reason for, the use of both or all three.

The inert tracer(s) must be transportable with the water of the boiler system (except of course the water discharged from the boiler as steam) and thus wholly water-soluble therein at the concentration it is used, under the temperature and pressure conditions to be encountered. Preferably the selected inert tracer(s) also meets the following criteria:

1. Be thermally stable and not decompose at the temperature within a boiler;
2. Be detectable on a continuous or semicontinuous basis and susceptible to concentration measurements that are accurate, repeatable and capable of being performed on feedwater and blowdown water;
3. Be substantially foreign to the chemical species that are normally present in the water of the boiler systems in which the inert tracer(s) may be used;
4. Be substantially impervious to interference from, or biasing by, the chemical species that are normally present in the water of the boiler systems in which the inert tracer(s) may be used;
5. Be substantially impervious to any of its own potential specific or selective losses from the water of the boiler system, including selective carry-over;
6. Be compatible with all treatment agents employed in the water of the boiler systems in which the inert tracer(s) may be used, and thus in no way reduce the efficacy thereof;
7. Be compatible with all components of its formulation and such compatibility preferably should endure despite the required concentrations of the tracer(s) and/or other components in such a formulation, and despite the storage and/or transportation conditions encountered; and
8. Be reasonably nontoxic and environmentally safe, not only within the environs of the water of the boiler system in which it may be used, but also upon discharge therefrom.

In preferred embodiment, the chemical compound(s) selected as an inert tracer(s) should not be one that is consumed or lost to the water of the boiler system, for instance due to degradation, deposition, complexation, or other phenomena, unless such consumption or loss is at a rate that is predictable. The inert tracer(s) used in the present invention is preferably substantially unconsumed in the use environment. An inert tracer(s) that is wholly inert in the water-system environment would not react with any of the components in the water of the boiler system to which it is added, would not degrade in the environment of the water of the boiler system, would be incapable of coupling and/or depositing in any manner within such boiler system and would not appreciably be effected by other system parameters such as metallurgical composition, heat changes or heat content. There are water-soluble inert tracer(s) that are wholly inert, or substantially inert, in the aqueous environments likely to be encountered in industrial boiler systems. Further, it is believed that an inert tracer(s) having a degree of inertness such that no more than 10 weight percent thereof is lost due to reaction, degradation, coupling and/or deposition during the time that elapses between its addition and its discharge as a blowdown component is sufficiently, or substantially, inert for the purpose of the present invention for most, if not all, tracer(s) monitorings.

Generally it is desirable to employ the least amount of inert and/or active tracer(s) that is practical for the circumstance, and the amount of the inert and/or active tracer(s) added to the water of the boiler system should be at least an amount effective for the determinations desired. When one the tracer is an active tracer the minimum amount thereof that is fed to the water of the boiler system should also be at least an amount effective for the desired activity thereof is such effective amount is greater than that required for the determinations desired. Seldom would an inert and/or active tracer(s) be deliberately fed to the water of a boiler system in an amount grossly in excess of the minimum effective amount because there generally would be no practical purpose in doing so that would justify the costs involved and any deleterious effects on the quality of the water of the boiler caused by the presence of the inert and/or active tracer(s) therein.

The amount of inert and/or active tracer(s) to be added to the water of the boiler system that is effective without being grossly excessive will vary with a variety of factors including, without limitation, the inert and/or active tracer(s) and monitoring method selected, the potential for background interference with the selected monitoring method, the magnitude of the expected inert and/or active tracer(s) concentration in the feedwater and/or blowdown, the monitoring mode (which generally would be an on-line continuous monitoring mode), and other similar factors. Generally the dosage of an inert tracer(s) to a water of the boiler system will be at least sufficient to provide a concentration of tracer(s) in the blowdown at steady state of at least about 0.1 ppb, and more commonly at least about 5 ppb or higher, up to about 100 or 200 ppm, in the blowdown.

By the terms "tracing" and "monitoring" are meant herein, unless expressly indicated otherwise, the determination of the concentration of the inert and/or active tracer(s) in the feedwater and/or blowdown. Such tracing/monitoring would seldom be conducted on a singular, intermittent or semi-continuous basis for the purpose of the present invention, but instead on a substantially continuous basis, and preferably the concentration determination is conducted on-site (at the site of the boiler system) to provide a rapid real-time determination.

The blowdown tracer(s) preferably is added to the water of the boiler system in known proportion to the feedwater, and preferably the blowdown tracer(s) is introduced into the boiler system together with the feedwater at a known and constant concentration therein.

The tracer(s) formulation, or "product", may be an aqueous solution or other substantially homogeneous admixture that disperses with reasonable rapidity in the system to which it is added. Since in most any instance the tracer(s) would be added to a boiler system as a component of a formulation, rather than as dry solid or neat liquid, the tracer(s)'s concentration may be correlated not to the numerical concentration value of the tracer(s) itself but instead to the concentration of a product, which in turn can be correlated to the concentration of the inert tracer(s) when and if such information is required.

Among the substantially boiler-system-inert fluorescent compounds are the mono-, di- and trisulfonated naphthalenes, including their water-soluble salts, particularly the various naphthalene mono- and disulfonic acid isomers, which are a preferred inert tracer(s) for use in the present invention. The naphthalene mono- and disulfonic acid isomers are water-soluble, generally available commercially and easily detectable and quantifiable by known fluorescence analysis techniques. Preferred naphthalene mono- and disulfonic acid isomers are the water-soluble salts of naphthalene sulfonic acid ("NSA"), such as 2-NSA, and naphthalene disulfonic acid ("NDSA" or "NDA"), for instance 1,5-NDSA. Many of these inert tracer(s) (mono-, di- and trisulfonated naphthalenes and mixtures thereof) are extremely compatible with the environments of most boiler systems. Among these preferred fluorescent tracers, 2-NSA and (1,5-NDSA) have been found to be thermally stable (substantially inert) at temperatures up to at least about 540 °C (1004 °F), for at least 24 hours at 285 °C (545 °F) and at pressures up to about 1,500 psig for time periods commensurate with commercial boiler holding times. Such inert fluorescent tracers have been found to carryover into the steam discharged from commercial boilers at concentrations of less than 500 ppt when present in the boiler waters at concentrations within the range of from about 5 to 10 ppm, and thus these tracers are not selectively carried over into the steam, and do not carry over into the steam in any appreciable amount. In addition, it has been found that the contribution to conductivity of the mono-, di- and trisulfonated naphthalenes is minimal at the ppb levels used for fluorescence determination in either the boiler feedwater or the blowdown.

Another group of inert fluorescent tracers that are preferred for use in the process of the present invention, particularly under pressures of no more than about 1,000 psi, are the various sulfonated derivatives of pyrene, such as 1,3,6,8-pyrene tetrasulfonic acid, and the various water-soluble salts of such sulfonated pyrene derivatives.

The inert and/or active tracer(s) is preferably selected from among those that are easily quantifiable by a fluorescence analysis method, a preferred analytical technique for the purposes of the present process. Other analysis methods not excluded for use in quantifying the inert and/or active tracer(s) are HPLC and fluorescence analysis combinations, which are described in more detail below.

An active tracer may be any chemical specie that is sufficiently water soluble and can be monitored in the feedwater and/or phosphate with sufficient ease for the purposes of the present invention. Active tracer chemicals may be lost to the boiler system due to both selective and nonselective mechanisms. In comparison, inert tracers are lost to the boiler system substantially only due to nonselective mechanisms such as removal as part of the blowdown. For the purposes of the present invention, an active tracer used as a feedwater and/or phosphate-feed tracer may be selectively lost to the boiler water in any amount by any mechanism, provided that such selective loss occurs downstream of the monitoring for such active tracer. An active tracer may have no role in boiler water chemistry other than its function as a feedwater and/or phosphate-feed tracer, but preferably it also functions as an operative chemical species within the boiler system.

An active tracer may be, for instance, a corrosion inhibitor. A corrosion control program usually depends on specific inhibitors to minimize the anodic or cathodic electrochemical corrosion reaction, or both. Among the various types of corrosion inhibitors are organic compounds, which act by adsorbing or chemisorbing as thin layers on metal surfaces to separate the water and metal. These materials form and maintain a dynamic barrier between the water and metal phases to prevent corrosion. One series of compounds applied to reduce copper and copper-alloy corrosion are aromatic organic corrosion inhibitors. This series of organic compounds, which includes mercaptobenzothiazole ("MBT"), benzotriazole ("BT"), butylbenzotriazole ("BBT"), tolyltriazole ("TT"), naphthotriazole ("NTA") and related compounds, react with the metal surface and form protective films on copper and copper alloys. These compounds are active corrosion inhibition treatment components and are referred to generally herein as copper corrosion inhibitors or corrosion inhibitors, or as aromatic azoles, and at times as triazoles or aromatic(thio)(tri)azoles. The conventional analytical procedure for analysis of copper corrosion inhibitors is a UV (ultraviolet light) photolysis/photometric method, and is well known to persons of ordinary skill in the art. This method, however, has the a number of limitations. It is not well suited for continuous monitoring and/or control. It provides results that are strongly dependent upon the operator's laboratory technique. It cannot distinguish the chemical structure of the aromatic(thio)(tri)azole that is present. Its observed response is non-linear with respect to aromatic(thio)(tri)azole dosage. It requires that the aromatic(thio)(tri) azoles be degraded with an ultraviolet lamp in the presence of a color-forming reagent. Therefore the preferred analytical technique for aromatic(thio)(tri)azoles when used as an active tracer in the process of the present invention is fluorescence emission spectroscopy discussed in more detail below. The use of fluorescence emission spectroscopy for the analysis of aromatic(thio)(tri)azoles is discussed in more detail in U.S. Patent Application Serial No. 07/872,624, filed on April 22, 1992, incorporated hereinto by reference.

Fluorescence Emission Spectroscopy

The detection and quantification of specific substances by fluorescence emission spectroscopy is founded upon the proportionality between the amount of emitted light and the amount of a fluoresced substance present. When energy in the form of light, including ultra violet and visible light, is directed into a sample cell, fluorescent substances therein will absorb the energy and then emit that energy as light having a longer wavelength than the absorbed light. A fluorescing molecule absorbs a photon resulting in the promotion of an electron from the ground energy state to an excited state. When the electron's excited state relaxes from a higher energy vibrationally-excited state to the lowest energy vibrationally-excited state, energy is lost in the form of heat. When the electron relaxes to the ground electronic state, light is emitted at a lower energy than that absorbed due to the heat-energy loss, and hence at a longer wavelength than the absorption. The amount of emitted light is determined by a photodetector. In practice, the light is directed into the sample cell through an optical light filter so that the light transmitted is of a known wavelength, which is referred to as the excitation wavelength and generally reported in nanometers ("nm"). The emitted light is similarly screened through a filter so that the amount of emitted light is measured at a known wavelength or a spectrum of wavelengths, which is referred to as the emission wavelength and generally also reported in nanometers. When the measurement of specific substances or categories of substances at low concentrations is desired or required, such as often is the case for the process of the present invention, the filters are set for a specific combination of excitation and emission wavelengths, selected for substantially optimum low-level measurements.

In general, the concentration of a tracer(s) can be determined from a comparison of a sample's emissions intensity to a calibration curve of the given tracer's concentration versus emissions, for the same set of excitation wavelength/ emission wavelengths. Such a concentration-by-comparison method by which the sensed emissions are converted to a concentration equivalent preferably is employed to determine concentrations of a tracer(s) that are within the concentration range over which a linear emission response is observed, and this concentration range is referred to herein as the "linear-emission-response concentration range". The linear-emission-response concentration range is to some extent dependent upon the specific tracer(s) and the excitation wavelength/emission wavelength set employed. At tracer concentrations higher than a given tracer(s)'s linear-emission-response concentration range, there is a negative deviation from ideal (linear) behavior, the degree of emission for a given concentration being less than predicted by a linear extrapolation. In such instances, the sample can be diluted by known factors until the concentration of the tracer (s) therein falls within the linear-emission-response concentration range. If the tracer(s) is present in the sample at only very low concentrations, there are techniques for concentrating the tracer(s) by known factors until its concentration falls within the linear-emission-response concentration range or is otherwise more readily measured, for instance by liquid-liquid extraction. Nonetheless, preferably a calibration curve over the linear-emission-response concentration range would be prepared or obtained before employing a given tracer(s), and preferably the tracer(s) would be added to the feedwater of the boiler system in an amount sufficient to provide a concentration of the tracer(s) in the feedwater and/or boiler that is within the linear-emission-response concentration range. Generally the linear-emission-response concentration range of a tracer(s) is sufficiently broad to readily estimate the amount of the tracer(s) that will be sufficient for this purpose. A linear-emission-response concentration range will most often extend through a concentration range from a concentration of "m" to a concentration of at least 10m.

A determination of the presence of a fluorescent tracer(s) and preferably the concentration thereof in the feedwater and/or blowdown from a boiler system can be made when the concentration of the tracer(s) in the feedwater and/or boiler is only several parts per million (ppm) or even parts per billion (ppb) for some of the tracer(s) that can be employed in the process of the present invention. In preferred embodiment, the amount of a fluorescent tracer(s) added to the feedwater and/or boiler system should be sufficient to provide a concentration of the tracer(s) in the feedwater and/or blowdown to be analyzed of from about 5 ppb to about 100 or 200 ppm, although the preferred inert tracers specifically mentioned herein need not be present in the sample analyzed in excess of about 5 or 7 ppm. Such analyses, that is, the measurements of the light emitted in response to the light transmitted to the feedwater and/or blowdown, can be made on-site, preferably on an almost instant and continuous basis, with simple portable equipment, such as the photodetector and screens described above.

At times it may be desired to employ a plurality of tracers for the reasons mentioned above and other reasons. For instance, it may be desired to use a plurality of tracers to confirm that neither is undergoing any tracer-specific loss or one tracer to detect a given variance and another for the detection of a different variance or other parameter. Such separate and distinct tracers can each be detected and quantified in a single feedwater and/or blowdown fraction despite both being fluorescent tracers if their respective wavelengths of emission do not interfere with one another. Thus concurrent analyses for multiple tracers is possible by selection of tracers having appropriate spectral characteristics. Preferably widely separated wavelengths of radiation should be used to excite each of the tracers and their fluorescent emissions should be observed and measured at widely separated emission wavelengths. A separate concentration calibration curve may be prepared or obtained for each tracer. In other words, more than one tracer can be employed, and then the presence and/or concentration of each such tracer in the feedwater and/or boiler system should

be determined using analytical parameters (particularly the excitation/emission wavelengths) effective for each such tracer, which analytical parameters preferably are sufficiently distinct to differentiate between measurements.

Fluorescence emission spectroscopy on a substantially continuous basis, at least over a given time period, is one of the preferred analytical techniques for the process of the present invention. It is one of the preferred analysis techniques for quantifying and determining the concentration of the tracer(s) in a boiler system and it is an analysis technique having significant advantages. Fluorescent chemical tracers and monitoring techniques are now known, for instance as disclosed in U.S. Patent No. 4,783,314, J. E. Hoots and B. E. Hunt, issued November 8, 1988, incorporated herein by reference, wherein inert fluorescent tracers are employed in combination with a fluorescence monitoring, such as the sodium salt of 2-naphthalenesulfonic acid.

When the tracer is 2-NSA, one of the water-soluble salts of naphthalene sulfonic acid ("NSA"), its concentration in the feedwater and/or blowdown from a boiler system can be fluorometrically measured by excitation at 277 nm and emission measurement at 334 nm, and the emissions observed referenced to a standard aqueous solution containing 0.5 ppm 2-NSA, as acid actives.

A Gilford Fluoro IV dual-monochromator spectrofluorometer can be used for a fluorometric analysis conducted on an intermittent basis and for on-line fluorescence monitoring, a portable fluorometer equipped with appropriate excitation and emission filters and a quartz flow through cell can be used, such as is commercially available from Turner Designs (Sunnyvale, California) Model Fluorometer 10 AU, which is mentioned above.

In general for most fluorescence emission spectroscopy methods having a reasonable degree of practicality, it is preferable to perform the analysis without isolating in any manner the tracer(s). Thus there may be some degree of background fluorescence in the feedwater and/or blowdown on which the fluorescence analysis is conducted, which background fluorescence may come from chemical compounds in the boiler system (including the feedwater system thereof) that are unrelated to the present process. In instances where the background fluorescence is low, the relative intensities (measured against a standard fluorescent compound at a standard concentration and assigned a relative intensity for instance 100) of the fluorescence of the tracer versus the background can be very high, for instance a ratio of 100/10 or 500/10 when certain combinations of excitation and emission wavelengths are employed even at low fluorescent compound concentrations, and such ratios would be representative of a "relative fluorescence" (under like conditions) of respectively 10 and 50. In preferred embodiment, the excitation/emission wavelengths and/or the amount of tracer employed are selected to provide a relative fluorescence of at least about 5 or 10 for the given background fluorescence anticipated.

For instance, for most feedwater and/or boiler water backgrounds, a compound that has a relative fluorescence of at least about 5 at a reasonable concentration is very suitable as a tracer(s). When there is or may be a specific chemical specie of reasonably high fluorescence in the background, the tracer(s) and/or the excitation and/or emission wavelengths often can be selected to nullify or at least minimize any interference of the tracer measurement(s) caused by the presence of such specie.

One method for the continuous on-stream monitoring of chemical tracers by fluorescence emission spectroscopy and other analysis methods is described in U.S. Patent No. 4,992,380, B. E. Moriarity, J. J. Hickey, W. H. Hoy, J. E. Hoots and D. A. Johnson, issued February 12, 1991, incorporated hereinto by reference.

### Combined HPLC-Fluorescence Analysis

The combination of high-pressure liquid chromatography ("HPLC") and fluorescence analyses of fluorescent tracers is a powerful tool for the present process, particularly when very low levels of the tracer(s) are used or the background fluorescence encountered would otherwise interfere with the efficacy of the fluorescence analysis. The HPLC-fluorescence analysis method allows the tracer compound(s) to be separated from the fluid matrix and then the tracer concentration(s) can be measured. The combination of HPLC-fluorescence analysis is particularly effective for measuring minute levels of the tracer(s) in highly contaminated fluids.

The HPLC method can also be effectively employed to separate a tracer compound(s) from a fluid matrix for the purposes of then employing a tracer-detection method(s) other than the fluorescence analysis, and such other tracer-detection methods include without limitation light absorbance, post-column derivatization, conductivity and the like, which methods are described in "Techniques in Liquid Chromatography", C. F. Simpson ed., John Wiley & Sons, New York, pp. 121-122, 1982, incorporated hereinto by reference, and "Standard Method For The Examination Of Water And Wastewater", 17th Edition, American Public Health Association, pp. 6-9 to 6-10, 1989, incorporated hereinto by reference.

Analytical techniques for quantifying the presence and/or concentration of a chemical specie without isolation thereof are within an evolving technology, and the above survey of reasonable analytical techniques for use in monitoring the tracer(s) in the process of the present invention may presently not even be exhaustive, and most likely techniques equivalent to the above for the purposes of the present invention will be developed in the future.

A tracer(s) may be selected for a given process based on a preference for one or more analytical techniques, or

an analytical technique may be selected for a given process based on a preference for one or more tracers.

As noted above, in preferred embodiment, the chemical compound(s) selected as the tracer(s) is soluble in the feedwater and/or boiler system to which it is added to the concentration value desired and is substantially stable in the environment thereof for the useful life expected of the tracer(s), particularly since it is desired not merely to detect the presence of some amount of the tracer(s), but also to determine the concentration thereof, or change in concentration. In preferred embodiment, the combination of the chemical compound(s) selected as the tracer(s) and the analytical technique selected for determining the presence of such tracer(s), permits such determination without isolation of the tracer(s), and more preferably should permit such determination on a continuous and/or on-line basis.

As discussed above, the phosphate-feed tracer in some preferred embodiments is added to the feedwater as a component of a formulated treatment product, particularly a formulated treatment product that also contains the phosphate being added to the feedwater. Formulating the tracer and the phosphate together is one technique that easily will provide a reliable correlation between the tracer feed rate and the phosphate feed rate. Such a formulated treatment product may also contain other treatment components, for instance dispersants and other treatment additives. For example, polymeric dispersants are often added to boilers to control iron deposits therein. Such dispersants include without limitation polyacrylic acid, polymethacrylic acid, acrylic acid/acrylamide copolymers, polyisopropenyl phosphonic acid and the water-soluble salts of such polymeric species, and these and other boiler additives are conveniently injected into the feedwater of a boiler system together with phosphate as a formulated treatment product. Such formulated treatment products routinely contain a diluent for the active components, which diluent can be water. The presence of such other boiler treatment additives in the formulation that contains one or more tracers is an embodiment, and at times a preferred embodiment of the present invention.

Although high pressure boilers and the requirements thereof are at times discussed above in relation to the present invention and pH/phosphate programs, neither the use of pH/phosphate programs nor the use of the present invention are limited to high pressure boilers. Instead, pH/phosphate programs and the present invention are also useful for boilers having lower operating pressures, for instance boilers having operating pressures of at least about 15 psi.

The above descriptions of boiler systems, pH/phosphate programs and other treatment additives for boilers are not exhaustive, and further descriptions are available in the literature in the boiler field, including in "The Chemical Treatment Of Boiler Water", James W. McCoy, pages 65-68, 81-87 and 131-139, 1981, 2nd printing 1984, Chemical Publishing Co., Inc., New York, N.Y., incorporated hereinto by reference, "The Nalco Guide To Boiler Failure Analysis", Robert D. Port and Harvey M. Herro, McGraw-Hill, Inc., New York, N.Y., incorporated hereinto by reference, "Steam - Its Generation And Use", 40th Edition, S. C. Stultz and J. B. Kitto ed., Babcock & Wilcox, a McDermott company, Barberton, Ohio, 1992, incorporated hereinto by reference, and "The Nalco Water Handbook", Second Edition, F. N. Kemmer ed., McGraw-Hill Company, New York, N.Y., 1988, particularly pp. 34.1-24.21, incorporated hereinto by reference.

The present invention is a method of controlling a pH/phosphate program in a boiler water system wherein

steam is generated from boiler water within a boiler and the steam is discharged from the boiler,
the boiler water contains impurities and the concentration of the impurities in the boiler water is reduced by discharging fractions of the boiler water as blowdown,
feedwater is fed to the boiler to replenish the water discharged from the boiler as steam and blowdown,
wherein the boiler has a concentration cycle value which is the value of the average concentration ("$C_F$") of an inert component in the blowdown at steady state divided by its concentration ("$C_I$") in the feedwater,
and phosphate and sodium hydroxide or other alkalinity source are fed to the boiler in amounts to attempt to provide a sodium to phosphate mole ratio in the boiler water within a target mole ratio range that is bounded by known $PO_4$/pH coordinates on a plot of orthophosphate concentration versus pH of a pH/phosphate program, comprising:

(1) determining the feed rate of the phosphate to the boiler by adding to a phosphate-feed stream containing the phosphate feeding to the boiler a phosphate-feed tracer in known proportion to the phosphate, determining the concentration of the phosphate-feed tracer in the phosphate-feed stream id correlating the phosphate-feed tracer concentration to the concentration of the phosphate;
(2) determining the boiler cycles value by adding an inert blowdown tracer to the boiler water at a known rate and determining the concentration of the blowdown tracer in the blowdown;
(3) determining the blowdown pH value;
(4) optionally determining the feed rate of the alkalinity source to the boiler by sampling a alkalinity source-feed stream containing the alkalinity source feeding to the boiler and determining the concentration of the alkalinity source in the alkalinity source-feed stream;
(5) providing the data defining at least the phosphate-feed rate value, the boiler cycles value, and the blowdown pH value to a control center;
(6) optionally maintaining the alkalinity source feed rate constant or providing the data defining the alkalinity

source feed rate to the control center;

wherein the control center directs at least one responsive adjustment of the phosphate feed to the boiler, the alkalinity source feed to the boiler, and/or the blowdown rate when a sodium to phosphate mole ratio in the boiler water strays from the target mole ratio,
wherein the responsive adjustment directed by the control center changes the sodium to phosphate mole ratio in the boiler water in the direction of regaining the target sodium to phosphate mole ratio in the boiler water.

In preferred embodiment, the phosphate is fed to the feedwater stream by a phosphate pump upstream of the boiler and the phosphate-feed stream is the feedwater stream downstream of the phosphate pump.

In some preferred embodiments, a solitary tracer is both the phosphate-feed tracer and the blowdown tracer. In further preferred embodiment, the solitary tracer is also a feedwater tracer and the concentration of the solitary tracer in the feedwater stream is correlated to the feed rate of the feedwater to the boiler.

In preferred embodiment, the phosphate-feed tracer is a fluorescent tracer and the concentration of the phosphate-feed tracer in the phosphate-feed stream is substantially continuously monitored on-line by sensing the fluorescent characteristic of the phosphate-feed tracer in the phosphate-feed stream. In preferred embodiment, the blowdown tracer is a fluorescent tracer and the boiler cycles is substantially continuously monitored on-line by sensing the fluorescent characteristic of the blowdown tracer in the blowdown.

In preferred embodiment, the phosphate-feed tracer and the blowdown tracer are distinct chemical species. In further preferred embodiment, the phosphate-feed tracer and the blowdown tracer are each fluorescent tracers and are distinct chemical species, wherein the phosphate-feed tracer and the blowdown tracer are each detected and quantified in a single feedwater and/or blowdown fraction by concurrent fluorescence analyses, and wherein sufficiently separated wavelengths of radiation are used to excite each of the phosphate-feed tracer and the blowdown tracer in the single fraction and the fluorescent emissions of the phosphate-feed tracer and the blowdown tracer are observed and measured at sufficiently separated emission wavelengths for the concurrent analysis. In further preferred embodiment, the phosphate-feed tracer and the blowdown tracer are distinct chemical species, wherein the phosphate-feed tracer and the blowdown tracer are each detected and quantified in a single feedwater and/or blowdown fraction by concurrent analyses, and wherein the concurrent analyses each employ a distinct concentration calibration curve for each respective distinct tracer.

In preferred embodiment, at least one of the phosphate-feed tracer and the blowdown tracer is a monosulfonated naphthalene, disulfonated naphthalene or trisulfonated naphthalene, or a water-soluble salt thereof. In more preferred embodiment, at least one of the phosphate-feed tracer and the blowdown tracer is a water-soluble salts of 2-naphthalene sulfonic acid or 1,5-naphthalene disulfonic acid.

In another preferred embodiment, at least one of the phosphate-feed tracer and the blowdown tracer is a sulfonated derivative of pyrene. In more preferred embodiment, at least one of the phosphate-feed tracer and the blowdown tracer is a 1,3,6,8-pyrene tetrasulfonic acid or water-soluble salt thereof.

In preferred embodiment, at least one of the phosphate-feed tracer and the blowdown tracer is a component of a formulated phosphate product.

In preferred embodiment, a solitary tracer is employed as a inert tracer to function as the phosphate-feed tracer, as a feedwater tracer, and as the blowdown tracer for boiler cycles determination purposes.

In preferred embodiment, a feedwater-tracer is added to the boiler in the feedwater, wherein the feedwater tracer and the phosphate-feed tracer are the same tracer or different tracers, and wherein at least one of the phosphate-feed tracer and the feedwater tracer is an active tracer that is a corrosion inhibitor. In more preferred embodiment, a feedwater-tracer is added to the boiler in the feedwater, wherein the feedwater tracer and the phosphate-feed tracer are the same tracer or different tracers, and wherein at least one of the phosphate-feed tracer and the feedwater tracer is an active tracer that is an aromatic azole.

In preferred embodiment, the phosphate-feed tracer is an inert tracer that is added to the water of the boiler system in an amount at least sufficient to provide a concentration of the inert tracer in the blowdown at steady state of at least about 0.1 ppm.

In preferred embodiment, at least one of the phosphate-feed tracer and the blowdown tracer is a fluorescent tracer and the fluorescent tracer is quantified to provide the phosphate feed rate value and/or the boiler cycles value by a combination of high-pressure liquid chromatography to separate the fluorescent tracer from a fluid matrix and fluorescence analyses of the fluorescent tracer.

In preferred embodiment, the phosphate-feed tracer is an active tracer, wherein the active tracer provides a performance benefit to the boiler water system downstream of the point at which the phosphate feed rate value is determined, and wherein the phosphate-feed tracer is added to the boiler in at least the amount effective to provide the performance benefit and at least in the amount effective for the determination of the phosphate feed rate value.

In certain preferred embodiments, the tracer selected is not a visible dye, that is, the tracer is a chemical specie

that does not have a strong absorption of electromagnetic radiation in the visible region, which extends from about 4000 to about 7000 Angstroms (from about 400 nm to about 700 nm). Such embodiments may be preferred when it is desirable to maintain a boiler water system free of color.

Unless expressly indicated otherwise herein, all properties of any chemical compounds, or compositions containing a plurality of chemical compounds, set forth herein are such property values as would be determined for such compounds, or compositions, within the temperature and substantially under atmospheric pressure of the use environment.

By deposits is meant herein material that forms and/or collects on surfaces of a boiler system or boiler equipment. By the terminology "__tracer concentration value" is meant herein the concentration of the tracer in the specified fluid in terms of weight of the tracer per unit volume of the fluid, or weight of the tracer per unit weight of the fluid, or some characteristic of the tracer that is proportional to its concentration in the fluid and can be correlated to a numerical value of the tracer concentration in the fluid (whether or not that correlation conversion is calculated, and can be a value of zero or substantially zero (for instance a value that is too low for detection). Thus the present process includes the detection of the absence of such chemical species, at least to the limitations of the analytical method employed.

Unless expressly indicated otherwise herein, the inclusion of a prefix or suffix in parenthesis designates the word with such prefix or suffix as an alternative. For instance, "specie(s)" means "specie and/or species", "determination(s) " means "determination and/or determinations", "tracer(s)" means "tracer and/or tracers", "technique(s)" means "technique and/or techniques", "chemical(s)" means "chemical and/or chemicals", "component(s)" means "component and/or components", and the like.

By "ppm", "ppb" and "ppt" is meant herein respectively "parts per million", "parts per billion" and "parts per trillion" wherein the "parts" are by weight.

Industrial Applicability of the Invention

The present invention is applicable to all industries employing a boiler system.

**Claims**

**1.** A method of controlling a pH/phosphate program in a boiler water system wherein steam is generated from boiler water within a boiler and said steam is discharged from said boiler, said boiler water contains impurities and the concentration of said impurities in said boiler water is reduced by discharging fractions of said boiler water as blowdown, feedwater is fed to said boiler to replenish said water discharged from said boiler as steam and blowdown, wherein said boiler has a concentration cycle value which is the value of the average concentration ("$C_F$") of an inert component in said blowdown at steady state divided by its concentration ("$C_I$") in said feedwater, and phosphate and optionally an alkalinity source are fed to said boiler in amounts to attempt to provide a sodium to phosphate mole ratio in said boiler water within a target mole ratio range that is bounded by known $PO_4$/pH coordinates on a plot of orthophosphate concentration versus pH of a pH/phosphate program, comprising:

(1) determining the feed rate of said phosphate to said boiler by adding to a phosphate-feed stream containing said phosphate feeding to said boiler a phosphate-feed tracer in known proportion to said phosphate, determining the concentration of said phosphate-feed tracer in said phosphate-feed stream and correlating said phosphate-feed tracer concentration to said concentration of said phosphate;
(2) determining said boiler cycles value by adding an inert blowdown tracer to said boiler water at a known rate and determining said concentration of said blowdown tracer in said blowdown;
(3) determining the blowdown pH value;
(4) optionally determining the feed rate of said alkalinity source to said boiler by sampling a alkalinity source-feed stream containing said alkalinity source feeding to said boiler and determining the concentration of said alkalinity source in said alkalinity source-feed stream;
(5) providing the data defining at least said phosphate-feed rate value, said boiler cycles value, and said blowdown pH value to a control center;
(6) optionally maintaining said alkalinity source feed rate constant or providing the data defining said alkalinity source feed rate to said control center;

wherein said control center directs at least one responsive adjustment of said phosphate feed to said boiler, said alkalinity source feed to said boiler, and/or said blowdown rate when a sodium to phosphate mole ratio in said boiler water strays from said target mole ratio,
wherein said responsive adjustment directed by said control center changes said sodium to phosphate mole ratio in said boiler water in the direction of regaining said target sodium to phosphate mole ratio in said boiler

water.

2. The method of Claim 1 wherein said phosphate is fed to said feedwater stream by a phosphate pump upstream of said boiler and said phosphate-feed stream is said feedwater stream downstream of said phosphate pump.

3. The method of Claim 1 or 2 wherein a solitary tracer is both said phosphate-feed tracer and said blowdown tracer.

4. The method of Claim 3 wherein said solitary tracer is also a feedwater tracer and the concentration of said solitary tracer in said feedwater stream is correlated to the feed rate of said feedwater to said boiler.

5. The method of any of claims 1 - 4 wherein said phosphate-feed tracer is a fluorescent tracer and said concentration of said phosphate-feed tracer in said phosphate-feed stream is substantially continuously monitored on-line by sensing the fluorescent characteristic of said phosphate-feed tracer in said phosphate-feed stream.

6. The method of any of claims 1 - 5 wherein said blowdown tracer is a fluorescent tracer and said boiler cycles is substantially continuously monitored on-line by sensing the fluorescent characteristic of said blowdown tracer in said blowdown.

7. The method of Claim 1 or 2 wherein said phosphate-feed tracer and said blowdown tracer are distinct chemical species.

8. The method of Claim 1 or 2 wherein said phosphate-feed tracer and said blowdown tracer are each fluorescent tracers and are distinct chemical species,

wherein said phosphate-feed tracer and said blowdown tracer are each detected and quantified in a single feedwater and/or blowdown fraction by concurrent fluorescence analyses, and
wherein sufficiently separated wavelengths of radiation are used to excite each of said phosphate-feed tracer and said blowdown tracer in said single fraction and the fluorescent emissions of said phosphate-feed tracer and said blowdown tracer are observed and measured at sufficiently separated emission wavelengths for said concurrent analysis.

9. The method of Claim 1 or 2 wherein said phosphate-feed tracer and said blowdown tracer are distinct chemical species,

wherein said phosphate-feed tracer and said blowdown tracer are each detected and quantified in a single feedwater and/or blowdown fraction by concurrent analyses, and
wherein said concurrent analyses each employ a distinct concentration calibration curve for each respective distinct tracer.

10. The method of any of Claims 1 - 9 wherein at least one of said phosphate-feed tracer and said blowdown tracer is a sulfonated naphthalene or a water-soluble salt thereof.

11. The method of any of Claims 1 - 10 wherein at least one of said phosphate-feed tracer and said blowdown tracer is a water-soluble salts of 2-naphthalene sulfonic acid or 1,5-naphthalene disulfonic acid.

12. The method of any of Claims 1 - 11 wherein at least one of said phosphate-feed tracer and said blowdown tracer is a sulfonated derivative of pyrene.

13. The method of any of Claims 1 - 12 wherein at least one of said phosphate-feed tracer and said blowdown tracer is a 1,3,6,8-pyrene tetrasulfonic acid or water-soluble salt thereof.

14. The method of any of Claims 1 - 13 wherein at least one of said phosphate-feed tracer and said blowdown tracer is a component of a formulated phosphate product wherein said formulated phosphate product optionally contains at least one boiler additive other than phosphate.

15. The method of any of Claims 1 - 3 wherein a solitary tracer is employed as an inert tracer to function as the phosphate-feed tracer, as a feedwater tracer, and as the blowdown tracer for boiler cycles determination purposes.

**16.** The method of any of Claims 1 - 15 wherein a feedwater-tracer is added to said boiler in said feedwater,

wherein said feedwater tracer and said phosphate-feed tracer are the same tracer or different tracers, and wherein at least one of said phosphate-feed tracer and said feedwater tracer is an active tracer that is a corrosion inhibitor.

**17.** The method of any of Claims 1 - 16 wherein a feedwater-tracer is added to said boiler in said feedwater,

wherein said feedwater tracer and said phosphate-feed tracer are the same tracer or different tracers, and wherein at least one of said phosphate-feed tracer and said feedwater tracer is an active tracer that is an aromatic azole.

**18.** The method of any of Claims 1 - 17 wherein said phosphate-feed tracer is an inert tracer that is added to the water of said boiler system in an amount at least sufficient to provide a concentration of said inert tracer in the blowdown at steady state of at least about 0.1 ppm.

**19.** The method of any of Claims 1 - 18 wherein at least one of said phosphate-feed tracer and said blowdown tracer is a fluorescent tracer and said fluorescent tracer is quantified to provide said phosphate feed rate value and/or said boiler cycles value by a combination of high-pressure liquid chromatography to separate said fluorescent tracer from a fluid matrix and fluorescence analyses of said fluorescent tracer.

**20.** The method of any of Claims 1 - 19 wherein said phosphate-feed tracer is an active tracer,

wherein said active tracer provides a performance benefit to said boiler water system downstream of the point at which said phosphate feed rate value is determined, and
wherein said phosphate-feed tracer is added to said boiler in at least in the amount effective to provide said performance benefit and at least in the amount effective for said determination of said phosphate feed rate value.

**Patentansprüche**

**1.** Verfahren zur Steuerung (Regelung) eines pH/Phosphat-Programms in einem Boiler-Wasser-System, bei dem Wasserdampf aus dem Boiler-Wasser innerhalb eines Boilers erzeugt wird und der genannte Wasserdampf aus dem Boiler ausgetragen wird, wobei das genannte Boiler-Wasser Verunreinigungen enthält und die Konzentration der genannten Verunreinigungen in dem genannten Boiler-Wasser durch Austragen von Fraktionen des genannten Boiler-Wassers als Blowdown vermindert wird, dem genannten Boiler Beschickungswasser zugeführt wird, um das aus dem Boiler in Form von Wasserdampf und Blowdown ausgetragene Wasser wieder zu ersetzen, wobei der genannte Boiler einen Konzentrationszykluswert aufweist, bei dem es sich um die Durchschnittskonzentration ("$C_F$") einer inerten Komponente in dem genannten Blowdown im Gleichgewichtszustand, dividiert durch ihre Konzentration ("$C_I$") in dem genannten Beschickungswasser handelt, und gegebenenfalls eine Alkalinitäts-Quelle dem genannten Boiler in solchen Mengen zugeführt wird, um ein Natrium/Phosphat-Molverhältnis in dem genannnten Boiler-Wasser innerhalb eines Ziel-Molverhältnis-Bereiches zu erzielen, der gegeben ist durch die bekannten $PO_4$/pH-Koordinaten in einem Diagramm, in dem die Orthophosphat-Konzentration gegen den pH-Wert eines pH/Phosphat-Programms aufgetragen ist, das umfaßt:

1) die Bestimmung der Einführungsrate des genannten Phosphats in den genannten Boiler durch Zugabe eines Phosphat-Beschickungs-Tracers zu einem Phosphat-Beschickungs-Strom, der die genannte Phosphat-Beschickung für den genannnten Boiler enthält, in einem bekannten Mengenverhältnis zu dem genannten Phosphat, die Bestimmung der Konzentration des genannnten Phosphat-Beschickungs-Tracers in dem genannten Phosphat-Beschickungs-Strom und das Inbeziehungsetzen (Korrelieren) der genannten Phosphat-Beschickungs-Tracer-Konzentration zu der genannten Konzentration des genannten Phosphats;

2) die Bestimmung des genannten Boilerzyklen-Wertes durch Zugabe eines inerten Blowdown-Tracers zu dem genannten Boiler-Wasser in einer bekannten Rate und die Bestimmung der genannten Konzentration des genannten Blowdown-Tracers in dem genannten Blowdown;

3) die Bestimmung des Blowdown-pH-Wertes;

4) gegebenenfalls die Bestimmung der Einführungsrate der genannten Alkalinitäts-Quelle in den genannten Boiler durch Entnahme einer Probe aus dem Alkalinitätsquellen-Beschickungsstrom, der die genannte Alkalinitätsquellen-Beschickung für den genannten Boiler enthält, und die Bestimmung der Konzentration der genannten Alkalinitätsquelle in dem genannten Alkalinitätsquellen-Beschickungsstrom;

5) die Aufgabe der Daten, die mindestens den Wert für die genannte Phosphat-beschickungsrate, den Boiler-Zyklen-Wert und den genannten Blowdown-pH-Wert definieren, auf ein Regel(Kontroll)-Zentrum;

6) gegebenenfalls die Konstanthaltung der genannten Alkalinitätsquellen-Beschickungsrate oder die Aufgabe der Daten, welche die genannte Alkalinitätsquellen-Beschickungsrate definieren, auf das genannte Regel (Kontroll)-Zentrum;

wobei das genannte Regel(Kontroll)-Zentrum mindestens eine dementsprechende Einstellung der genannten Phosphat-Einführung in den genannten Boiler, der genannten Einführung der Alkalinitätsquelle in den genannten Boiler und/oder der genannten Blowdown-Rate vornimmt, wenn das Natrium/Phosphat-Molverhältnis in dem genannten Boiler-Wasser von dem genannten Ziel-Molverhältnis abweicht, und

wobei die genannte dementsprechende Einstellung, die von dem genannten Regel(Kontroll)-Zentrum vorgenommen wird, das genannte Natrium/Phosphat-Molverhältnis in dem Boiler-Wasser in Richtung auf die Wiedereinstellung des Ziel-Natrium/Phosphat-Molverhältnisses in dem genannten Boiler-Wasser ändert.

**2.** Verfahren nach Anspruch 1, worin das genannte Phosphat dem genannten Beschickungswasser-Strom mittels einer Phosphat-Pumpe stromaufwärts von dem genannten Boiler zugeführt wird und der genannte Phosphat-Beschickungsstrom der genannte Beschickungswasser-Strom stromabwärts von der genannten Phosphat-Pumpe ist.

**3.** Verfahren nach Anspruch 1 oder 2, worin ein einzelner Tracer sowohl den genannten Phosphat-Beschickungs-Tracer als auch den genannten Blowdown-Tracer darstellt.

**4.** Verfahren nach Anspruch 3, worin der genannte einzelne Tracer auch ein Beschickungswasser-Tracer ist und die Konzentration des genannten einzelnen Tracers in dem Beschickungswasser-Strom in Beziehung steht (korreliert) zu der Einführungsrate des genannten Beschickungswassers in den genannten Boiler.

**5.** Verfahren nach einem der Ansprüche 1 bis 4, worin der genannte Phosphat-Beschickungs-Tracer ein fluoreszierender Tracer ist und die genannte Konzentration des genannten Phosphat-Beschickungs-Tracers in dem genannten Phosphat-Beschickungs-Strom im wesentlichen kontinuierlich online überwacht wird durch Bestimmung der Fluoreszenz-Charakteristik des genannten Phosphat-Beschickungs-Tracers in dem genannten Phosphat-Beschickungs-Strom.

**6.** Verfahren nach einem der Ansprüche 1 bis 5, worin der genannte Blowdown-Tracer ein fluoreszierender Tracer ist und die genannten Boiler-Zyklen im wesentlichen kontinuierlich online überwacht werden durch Bestimmung der Fluoreszenz-Charakteristik des genannten Blowdown-Tracers in dem genannten Blowdown.

**7.** Verfahren nach Anspruch 1 oder 2, worin der genannte Phosphat-Beschickungs-Tracer und der genannte Blowdown-Tracer unterschiedliche chemische Verbindungen sind.

**8.** Verfahren nach Anspruch 1 oder 2, worin der genannte Phosphat-Beschickungs-Tracer und der genannte Blowdown-Tracer jeweils fluroeszierende Tracer und unterschiedliche chemische Verbindungen sind,

wobei der genannte Phosphat-Beschickungs-Tracer und der genannte Blowdown-Tracer jeweils nachgewiesen und quantitativ bestimmt werden in einer einzigen Beschickungswasser- und/oder Blowdown-Fraktion durch gleichzeitige Fluoreszenzanalysen und

worin eine Strahlung mit ausreichend verschiedenen Wellenlängen verwendet wird, um sowohl den genannten Phosphat-Beschickungs-Tracer als auch den genannten Blowdown-Tracer in der genannten einzelnen Fraktion zu erregen und die Fluoreszenz-Emissionen des genannten Phosphat-Beschickungs-Tracers und des genannten Blowdown-Tracers beobachtet und gemessen werden bei ausreichend voneinander getrennten -Emissions-Wellenlängen zur Durchführung der genannten gleichzeitigen Analyse.

**9.** Verfahren nach Anspruch 1 oder 2, worin der genannte Phosphat-Beschickungs-Tracer und der genannte Blowdown-Tracer unterschiedliche chemische Verbindungen sind,

wobei der genannte Phosphat-Beschickungs-Tracer und der genannte Blowdown-Tracer jeweils in einer einzelnen Beschickungswasser- und/oder Blowdown-Fraktion nachgewiesen und durch gleichzeitige Analysen und quantitativ bestimmt werden und

wobei in den genannten gleichzeitigen Analysen jeweils eine andere Konzentrationseichkurve für den jeweiligen unterschiedlichen Tracer verwendet wird.

**10.** Verfahren nach einem der Ansprüche 1 bis 9, worin mindestens einer aus der Gruppe des genannten Phosphat-Beschickungs-Tracers und des genannten Blowdown-Tracers ein sulfoniertes Naphthalin oder ein wasserlösliches Salz davon ist.

**11.** Verfahren nach einem der Ansprüche 1 bis 10, worin mindestens einer aus der Gruppe des genannten Phosphat-Beschickungs-Tracers und des genannten Blowdown-Tracers ein wasserlösliches Salz von 2-Naphthalinsulfonsäure oder 1,5-Naphthalindisulfonsäure ist.

**12.** Verfahren nach einem der Ansprüche 1 bis 11, worin mindestens einer aus der Gruppe des genannten Phosphat-Beschickungs-Tracers und des genannten Blowdown-Tracers ein sulfoniertes Derivat von Pyren ist.

**13.** Verfahren nach einem der Ansprüche 1 bis 12, worin mindestens einer aus der Gruppe des genannten Phosphat-Beschickungs-Tracers und des genannten Blowdown-Tracers eine 1,3,6,8-Pyren-tetrasulfonsäure oder ein wasserlösliches Salz derselben ist.

**14.** Verfahren nach einem der Ansprüche 1 bis 13, worin mindestens einer aus der Gruppe des genannten Phosphat-Beschickungs-Tracers und des genannten Blowdown-Tracers eine Komponente eines formulierten Phosphat-Produkts ist, wobei das genannte formulierte Phosphat-Produkt gegebenenfalls mindestens einen von einem Phosphat verschiedenen Boiler-Zusatz enthält.

**15.** Verfahren nach einem der Ansprüche 1 bis 3, worin ein einzelner Tracer als inerter Tracer verwendet wird, der als Phosphat-Beschickungs-Tracer, als Beschikkungswasser-Tracer und als Blowdown-Tracer zur Bestimmung der Boilerzyklen fungiert.

**16.** Verfahren nach einem der Ansprüche 1 bis 15, worin ein Beschickungswasser-Tracer dem genannten Boiler in dem genannten Beschickungswasser zugesetzt wird,

wobei der genannte Beschickungswasser-Tracer und der genannte Phosphat-Beschickungs-Tracer die gleichen Tracer oder unterschiedliche Tracer sind und

wobei mindestens einer aus der Gruppe des genannten Phosphat-Beschickungs-Tracers und des genannten Beschickungswasser-Tracers ein aktiver Tracer ist, der einen Korrosionsinhibitor darstellt.

**17.** Verfahren nach einem der Ansprüche 1 bis 16, worin ein Beschickungswasser-Tracer dem genannten Boiler in dem genannten Beschickungswasser zugesetzt wird,

wobei der genannte Beschickungswasser-Tracer und der genannte Phosphat-Beschickungs-Tracer die gleichen Tracer oder unterschiedliche Tracer sind und

wobei mindestens einer aus der Gruppe des genannten Phosphat-Beschickungs-Tracers und des Beschikkungswasser-Tracers ein aktiver Tracer ist, bei dem es sich um ein aromatisches Azol handelt.

**18.** Verfahren nach einem der Ansprüche 1 bis 17, worin der genannte Phosphat-Beschickungs-Tracer ein inerter Tracer ist, der dem Wasser des genannten Boiler-Systems in einer Menge zugesetzt wird, die mindestens ausreicht, um eine Konzentration an dem genannten inerten Tracer in dem Blowdown im Gleichgewichtszustand von mindestens etwa 0,1 ppm zu ergeben.

**19.** Verfahren nach einem der Ansprüche 1 bis 18, wobei mindestens einer aus der Gruppe des genannten Phosphat-Beschickungs-Tracers und des genannten Blowdown-Tracers ein fluoreszierender Tracer ist und der genannte fluoreszierende Tracer quantitativ so festgelegt wird, daß er den genannten Phosphat-Beschickungsraten-Wert und/oder den genannten Boilerzyklen-Wert ergibt, durch eine Kombination von Hochdruck-Flüssigkeitschromatographie, um den genannten fluoreszierenden Tracer von einer Flüssigkeitsmatrix abzutrennen, und Fluoreszenzanalysen des genannten Fluoreszenz-Tracers.

**20.** Verfahren nach einem der Ansprüche 1 bis 19, worin der genannte Phosphat-Beschickungs-Tracer ein aktiver Tracer ist,

wobei der genannte aktive Tracer dem Boilerwasser-System stromabwärts von dem Punkt, an dem der Wert für die Phosphat-Beschickungsrate bestimmt wird, einen Leistungsvorteil ergibt, und

wobei der genannte Phosphat-Beschickungs-Tracer dem genannten Boiler zugegeben wird in mindestens der Menge, die wirksam ist, um den genannten Leistungsvorteil zu erzielen, und mindestens in der Menge, die wirksam ist zur Bestimmung des Wertes der genannten Phosphat-Beschickungsrate.

## Revendications

**1.** Procédé pour le contrôle d'un programme de pH/phosphate dans un système d'eau de chaudière dans lequel de la vapeur d'eau est produite de l'eau de chaudière à l'intérieur d'une chaudière et ladite vapeur est expulsée de ladite chaudière, ladite eau de chaudière contenant des impuretés et la concentration desdites impuretés dans l'eau de chaudière étant réduite par expulsion de fractions de ladite eau de chaudière en tant que "blowdown", de l'eau d'alimentation étant amenée à ladite chaudière pour remplacer l'eau extraite en forme de vapeur et de "blowdown", ladite chaudière ayant une valeur de cycle de concentration qui est la valeur de la concentration moyenne ("$C_F$") d'un composant inerte dans ledit blowdown à l'état d'équilibre divisée par sa concentration ("$C_I$") dans ladite eau d'alimentation, et du phosphate et éventuellement une source d'alcalinité étant amenés à la chaudière dans des quantités telles qu'un rapport molaire sodium/phosphate est obtenu dans ladite chaudière dans une gamme de rapport cible-mole qui est donnée par les coordonnées connues de $PO_4$/pH dans un diagramme dans lequel la concentration d'orthophosphate est inscrite contre le pH d'un programme pH/phosphate, comprenant:

1) la détermination de la vitesse d'alimentation dudit phosphate dans ladite chaudière par addition d'un traceur d'alimentation de phosphate à un courant d'alimentation de phosphate contenant ladite alimentation de phosphate dans ladite chaudière dans une proportion connue audit phosphate, la détermination de la concentration dudit traceur d'alimentation de phosphate dans ledit courant d'alimentation de phosphate et la corrélation de ladite concentration de traceur d'alimentation de phosphate à ladite concentration dudit phosphate,
2) la détermination de ladite valeur de cycle de chaudière par addition d'un traceur inerte de blowdown à ladite eau de chaudière à une vitesse connue et la détermination de ladite concentration dudit traceur connu de blowdown dans ledit blowdown;
3) la détermination du pH du blowdown,
4) le cas échéant, la détermination de la vitesse d'alimentation de ladite source d'alcalinité dans ladite chaudière par échantillonnage du courant d'alimentation de source d'alcalinité qui contient ladite alimentation de source d'alcalinité pour ladite chaudière, et la détermination de la concentration de ladite source d'alcalinité dans ledit courant d'alimentation de la source d'alcalinité,
5) la fourniture des données définissant au moins la valeur de ladite vitesse d'alimentation du phosphate, ladite valeur de cycles de chaudière et ledit pH de blowdown à un centre de contrôle,
6) le cas échéant, le maintien à valeur constante de ladite vitesse d'alimentation de source d'alcalinité ou la fourniture des données définissant ladite vitesse d'alimentation de source d'alcalinité audit centre de contrôle,

ledit centre de contrôle exécutant au moins un ajustage correspondant de ladite alimentation de phosphate dans ladite chaudière, de ladite alimentation de la source d'alcalinité dans ladite chaudière et/ou de ladite vitesse de blowdown quand un rapport molaire de sodium/phosphate dans ladite eau de chaudière diffère dudit rapport cible-mole,
ledit ajustement correspondant effectué par ledit centre de contrôle changeant ledit rapport molaire sodium/phosphate dans ladite eau de chaudière en direction de la remise dudit rapport molaire sodium/phosphate dans ladite eau de chaudière.

2. Procédé selon la revendication 1, dans lequel ledit phosphate est amené audit courant d'eau d'alimentation à l'aide d'une pompe à phosphate en amont de ladite chaudière et dans lequel ledit courant d'alimentation de phosphate est ledit courant d'eau d'alimentation en aval de ladite pompe à phosphate.

3. Procédé selon la revendication 1 ou 2, dans lequel un traceur solitaire représente aussi bien le traceur d'alimentation de phosphate que le traceur de blowdown.

4. Procédé selon la revendication 3, dans lequel ledit traceur solitaire est aussi un traceur d'eau d'alimentation et la concentration dudit traceur solitaire dans le courant d'eau d'alimentation est correlé à la vitesse d'alimentation de ladite eau d'alimentation dans ladite chaudière.

5. Procédé selon l'une des revendications 1 à 4, dans lequel ledit traceur d'alimentation de phosphate est un traceur fluorescent et ladite concentration dudit traceur d'alimentation de phosphate dans ledit courant d'alimentation de phosphate est surveillée essentiellement en continu "on-line" par détermination de la caractéristique fluorescente dudit traceur d'alimentation de phosphate dans ledit courant d'alimentation de phosphate.

6. Procédé selon l'une des revendications 1 à 5, dans lequel ledit traceur de blowdown est un traceur fluorescent et lesdits cycles de chaudière sont surveillés essentiellement en continu on-line par détermination de la caractéristique fluorescente dudit traceur de blowdown dans ledit blowdown.

7. Procédé selon la revendication 1 ou 2, dans lequel ledit traceur d'alimentation de phosphate et ledit traceur de blowdown sont des composés chimiques différents.

8. Procédé selon la revendication 1 ou 2, dans lequel ledit traceur d'alimentation de phosphate et ledit traceur de blowdown sont respectivement des traceurs fluorescents et des composés chimiques différents,

   dans lequel ledit traceur d'alimentation de phosphate et ledit traceur de blowdown sont respectivement décelés et déterminés en quantité dans une seule fraction d'eau d'alimentation et/ou fraction de blowdown par analyses de fluorescence simultanées et
   dans lequel un rayonnement avec des longueurs d'onde suffisamment différentes pour exciter aussi bien ledit traceur d'alimentation de phosphate que ledit traceur de blowdown dans ladite fraction individuelle et les émissions de fluorescence dudit traceur d'alimentation de phosphate et dudit traceur de blowdown sont observées et mesurées à longueurs d'onde d'émission suffisamment éloignées pour la réalisation de ladite analyse simultanée.

9. Procédé selon la revendication 1 ou 2, dans lequel ledit traceur d'alimentation de phosphate et ledit traceur de blowdown sont des composés chimiques différents,

   dans lequel ledit traceur d'alimentation de phosphate et ledit traceur de blowdown sont respectivement décelés et déterminés en quantité dans une seule fraction d'eau d'alimentation et/ou de blowdown par des analyses simultanées,
   et dans lequel une courbe de calibration de concentration différente est utilisée dans chaque analyse simultanée pour les traceurs individuels.

10. Procédé selon l'une des revendications 1 à 9, dans lequel au moins un traceur des traceurs d'alimentation de phosphate et de blowdown est un naphtaline sulfoné ou son sel soluble à l'eau.

11. Procédé selon l'une des revendications 1 à 10, dans lequel au moins un traceur des traceurs d'alimentation de phosphate et de blowdown est un sel soluble à l'eau d'acide sulfonée de 2-naphthaline ou d'acide disulfonée de 1,5-naphthaline.

12. Procédé selon l'une des revendications 1 à 11, dans lequel au moins un traceur des traceurs d'alimentation de phosphate et de blowdown est un dérivé sulfoné de pyrène.

13. Procédé selon l'une des revendications 1 à 12, dans lequel au moins un traceur des traceurs d'alimentation de phosphate et de blowdown est un acide tétrasulfonée de 1,3,6,8-pyrène ou son sel soluble à l'eau.

14. Procédé selon l'une des revendications 1 à 13, dans lequel au moins un traceur des traceurs d'alimentation de

phosphate et de blowdown est un composant d'un produit de phosphate formulé, dans lequel ledit produit de phosphate formulé contient le cas échéant au moins un supplément à la chaudière différent du phosphate.

**15.** Procédé selon l'une des revendications 1 à 3, dans lequel un traceur solitaire est utilisé en tant que traceur inerte pour fonctionner comme un traceur d'alimentation de phosphate, comme un traceur d'eau d'alimentation et comme un traceur de blowdown pour déterminer les cycles de chaudière.

**16.** Procédé selon l'une des revendications 1 à 15, dans lequel un traceur d'eau d'alimentation est ajouté à ladite chaudière dans ladite eau d'alimentation,

dans lequel ledit traceur d'eau d'alimentation et ledit traceur d'alimentation de phosphate sont le même traceur ou ils sont des traceurs différents et
dans lequel au moins un des traceurs du groupe de traceur d'alimentation de phosphate et de traceur d'eau d'alimentation est un traceur actif qui représente un inhibiteur de corrosion.

**17.** Procédé selon l'une des revendications 1 à 16, dans lequel un traceur d'eau d'alimentation est ajouté à ladite chaudière dans ladite eau d'alimentation,

dans lequel ledit traceur d'eau d'alimentation et ledit traceur d'alimentation de phosphate est le même ou ils sont traceurs différents et
dans lequel au moins un des traceurs du groupe de traceur d'alimentation de phosphate et de traceur d'eau d'alimentation est un traceur actif qui est un azole aromatique.

**18.** Procédé selon l'une des revendications 1 à 17, dans lequel ledit traceur d'alimentation de phosphate est un traceur inerte qui est ajouté à l'eau dudit système de chaudière dans une quantité qui est au moins suffisante pour obtenir une concentration dudit traceur inerte dans le blowdown dans un état d'équilibre d'au moins environ 0,1 ppm.

**19.** Procédé selon l'une des revendications 1 à 18, dans lequel au moins un des traceurs du groupe de traceur d'alimentation de phosphate et de traceur de blowdown est un traceur fluorescent et dans lequel ledit traceur fluorescent est présent en une quantité telle qu'il délivre ladite valeur de vitesse d'alimentation de phosphate et/ou ladite valeur de cycles de chaudière par une combinaison de chromatographie liquide haute pression pour séparer ledit traceur fluorescent d'une matrice de liquide, et par analyses de fluorescence dudit traceur fluorescent.

**20.** Procédé selon l'une des revendications 1 à 19, dans lequel ledit traceur d'alimentation de phosphate est un traceur actif,

dans lequel ledit traceur actif délivre un avantage de performance en aval du point où la valeur de la vitesse d'alimentation de phosphate est déterminée et
dans lequel ledit traceur d'alimentation de phosphate est ajouté à ladite chaudière dans au moins la quantité qui est effective pour obtenir ledit avantage de performance et au moins dans la quantité qui est effective pour la détermination de la valeur de ladite vitesse d'alimentation de phosphate.

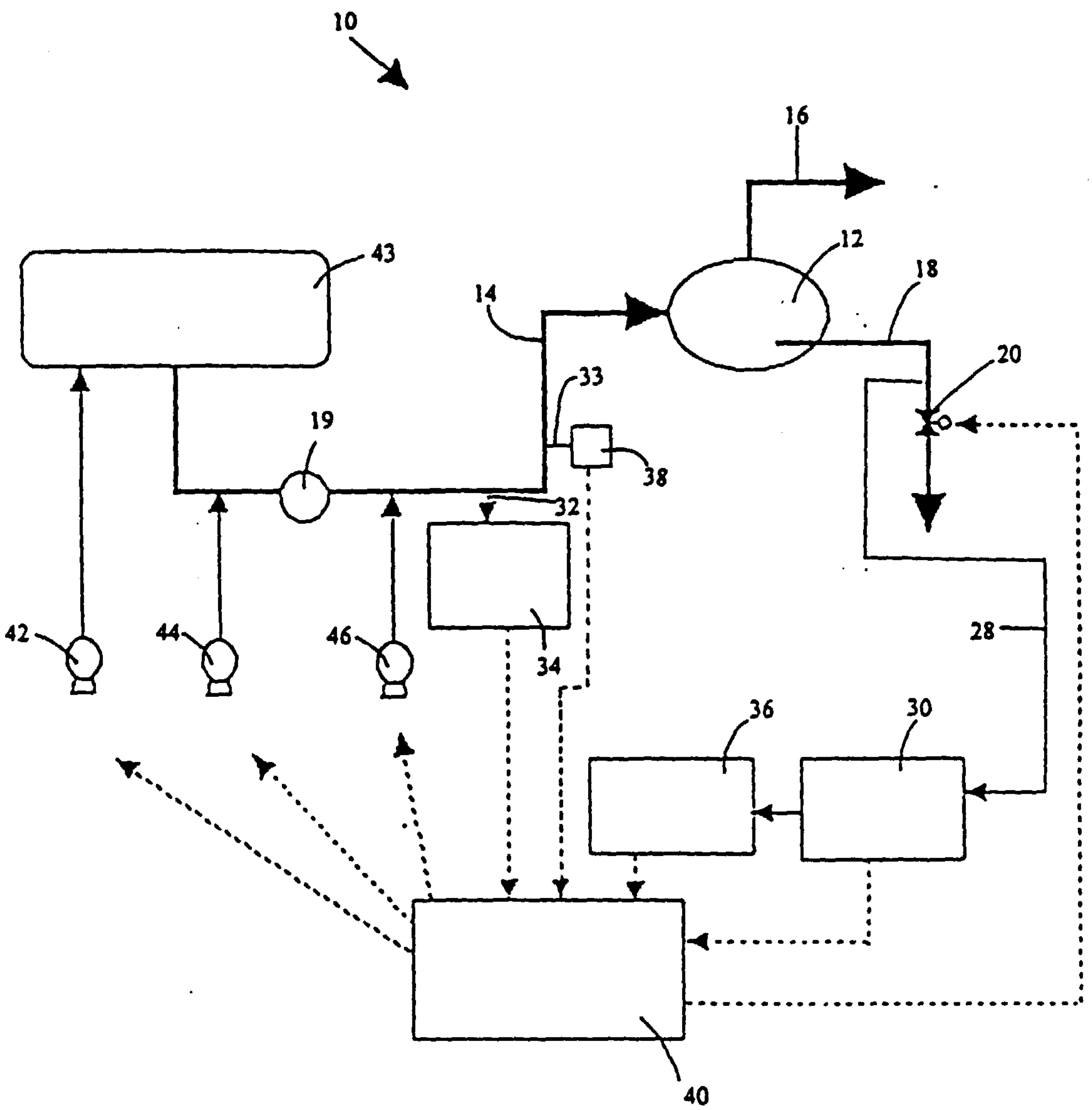


FIGURE 1

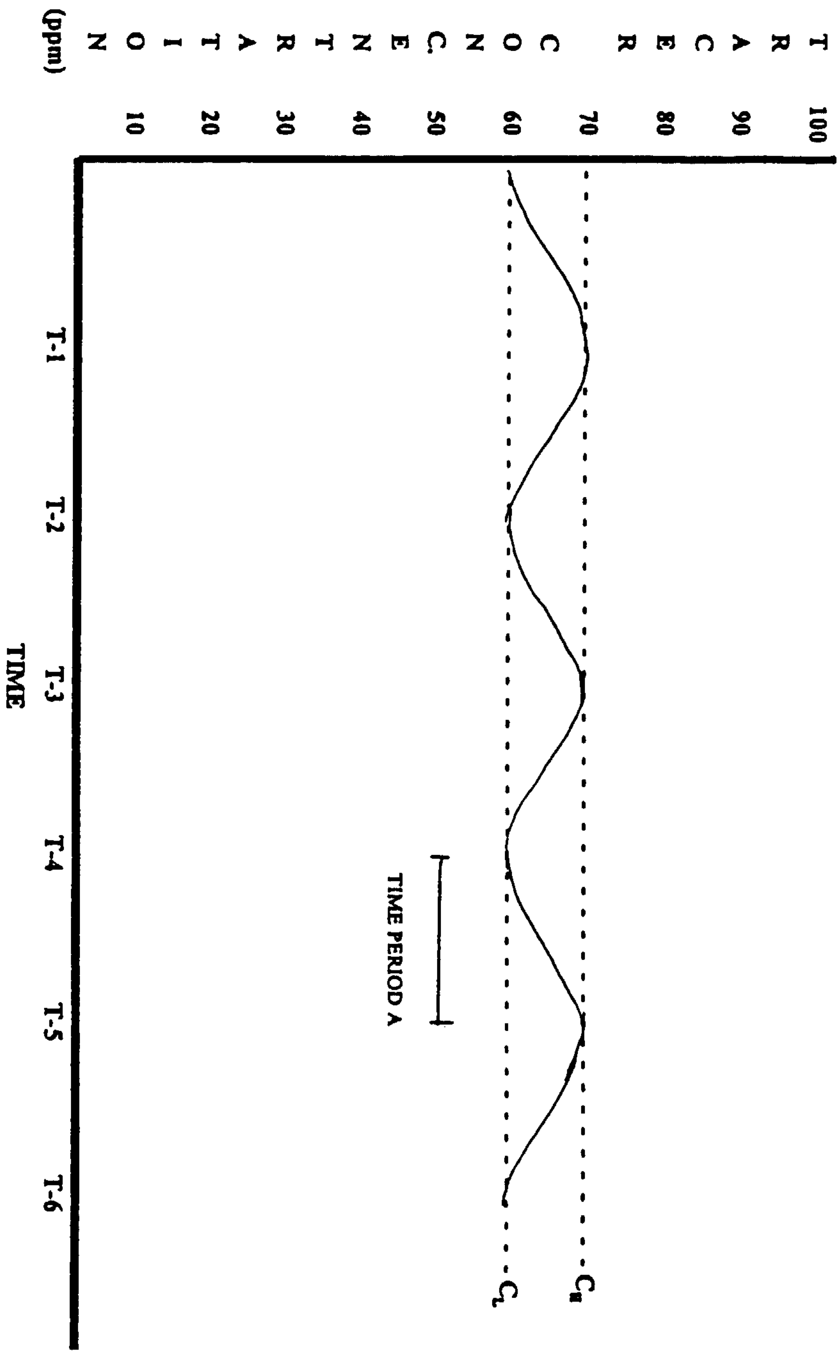
TRACER CONCENTRATION (ppm)
100
90
80
70
60
50
40
30
20
10
T-1
T-2
T-3
T-4
T-5
T-6
TIME
TIME PERIOD A
$C_H$
$C_L$

FIGURE 2

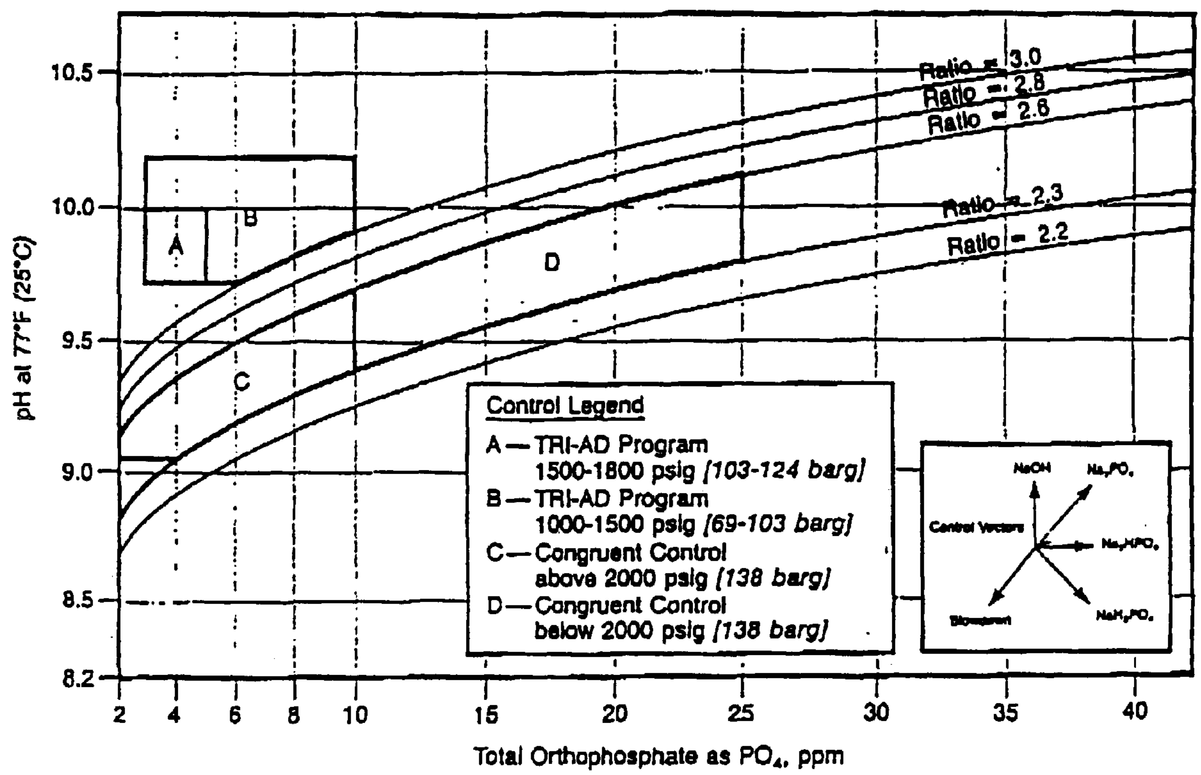
10.5
10.0
9.5
9.0
8.5
8.2
pH at 77°F (25°C)
2
4
6
8
10
15
20
25
30
35
40
Total Orthophosphate as $PO_4$, ppm
Ratio = 3.0
Ratio = 2.8
Ratio = 2.6
Ratio = 2.3
Ratio = 2.2
A
B
C
D
Control Legend
A — TRI-AD Program
1500-1800 psig [103-124 barg]
B — TRI-AD Program
1000-1500 psig [69-103 barg]
C — Congruent Control
above 2000 psig [138 barg]
D — Congruent Control
below 2000 psig [138 barg]
Control Vectors
NaOH
$Na_3PO_4$
$Na_2HPO_4$
$NaH_2PO_4$

FIGURE 3